# FASCICULE DE BREVET EUROPEEN

(11) **EP 1 071 798 B1**
(45) Date de publication et mention de la délivrance du brevet: **25.01.2006**
(21) Numéro de dépôt: 99913408.3
(22) Date de dépôt: 14.04.1999
(51) Int. Cl.: C12N 15/71, C12N 9/88

(54) **CONSTRUCTIONS POUR L'EXPRESSION CONTROLEE DE GENES RECOMBINANTS DANS DES CELLULES PROCARYOTES**
VEKTOREN ZUR KONTROLLIERTER EXPRESSION VON REKOMBINANTEN GENEN IN PROKARYONTISCHEN ZELLEN
CONSTRUCTS FOR CONTROLLED EXPRESSION OF RECOMBINANT PROTEINS IN PROKARYOTIC CELLS

(30) Priorité: 14.04.1998 FR 9804638
(43) Date de publication de la demande: 31.01.2001
(73) Titulaire: PIERRE FABRE MEDICAMENT, 92100 Boulogne-Billancourt (FR)
(72) Inventeur: CHEVALET, Laurent, F-74100 Annemasse (FR); ROBERT, Alain, F-74100 Annemasse (FR); BONNEFOY, Jean-Yves, F-74250 Le Sappey (FR); NGUYEN, Thien, Ngoc, F-74160 Saint-Julien-en-Genevois (FR)
(74) Mandataire: Ahner, Francis
(86) Numéro de dépôt international: PCT/FR1999/000874
(87) Numéro de publication internationale: WO 1999/053080

(56) Documents cités:
- EP-A- 0 293 207
- US-A- 5 416 008
- YANSURA D ET AL: "Use of Escherichia coli trp promoter for direct expression of proteins" METHODS IN ENZYMOLOGY, vol. 185, 1990, pages 54-60, XP002088409 cité dans la demande
- YANOFSKY C ET AL: "Some novel transcription attenuation mechanisms used by bacteria" BIOCHIMIE, vol. 78, no. 11-12, 1996, pages 1017-1024, XP002088410
- WARNE S ET AL: "Use of a modified Escherichia coli trpR gene to obtain tight regulation of high-copy-number expression vectors" GENE, vol. 46, 1986, pages 103-112, XP002088411

## Description

L'invention comprend une nouvelle construction pour l'expression d'un gène codant pour une protéine recombinante d'intérêt placé sous le contrôle du promoteur de l'opéron tryptophane Ptrp dans une cellule hôte procaryote, caractérisée en ce que la construction comprend une séquence nucléique capable d'inactiver le gène codant pour une tryptophanase TnaA lorsque ladite séquence nucléique est introduite dans ladite cellule hôte et d'une séquence d'un promoteur suivie en 3' d'une séquence nucléique codant pour une molécule de nature ribonucléotidique ou protéique agissant négativement sur le promoteur Ptrp ou son produit de transcription, des vecteurs contenant ladite construction et les cellules hôtes transformées par lesdits vecteurs. L'invention a également pour objet les procédés de production desdites protéines recombinantes utilisant ces nouvelles constructions.

La présente invention s'applique de manière générale à la production de protéines ou de polypeptides recombinants par des méthodes dites de l'ADN recombinant. Plus particulièrement, la présente invention concerne la production de protéines ou de polypeptides recombinants par des cellules hôtes transformées de type bactérien et dont l'expression est dirigée ou est sous le contrôle du promoteur / opérateur Ptrp de l'opéron tryptophane (Nichols & Yanofsky, 1983).

*Escherichia coli (E coli*) est l'organisme le plus couramment utilisé et le mieux caractérisé dans un but de production de protéines recombinantes. Différents systèmes d'expression sont employés dans E. *coli* et, parmi eux, le promoteur Ptrp de l'opéron tryptophane est considéré comme l'un des plus forts (Yansura & Bass, 1997).

Cependant, tous les gènes recombinants ne sont pas exprimés avec la même efficacité par E. *coli*. Il a été décrit et observé que l'accumulation d'une protéine recombinante produite lors de la culture de cellules hôtes transformées pouvait rapidement conduire à une instabilité plasmidique, à une diminution voire même un arrêt de la croissance cellulaire et à une diminution du rendement global en produit recombinant. Dans ce cas, il est important de disposer d'un système d'expression contrôlée et régulée permettant de diviser le procédé de production en deux phases, une première dite de croissance cellulaire où l'activité du promoteur est minimale, suivie d'une phase dite d'induction ou de dérépression privilégiant l'expression et l'accumulation de la protéine recombinante.

Ptrp, le promoteur de l'opéron tryptophane d'*E*. *coli,* est adapté à la production de protéines recombinantes du fait de son caractère inductible. La répression au niveau de l'opérateur de Ptrp est assurée par le produit du gène régulateur *trp*R lorsque ce produit, également nommé aporépresseur trp, est lié au tryptophane (co-répresseur). L'absence de tryptophane rend la protéine TrpR incapable de se lier à l'opérateur, provoquant ainsi une dérépression de l'opéron tryptophane. Divers exemples d'expression de gènes hétérologues sous le contrôle de Ptrp montrent que la fuite d'expression y est trop importante pour permettre la production, dans des conditions satisfaisantes de protéines recombinantes, notamment celles qui sont toxiques pour la cellule (Yansura et Henner, 1990).

La protéine régulatrice TrpR est soumise à un mécanisme d'auto-régulation (Kelley & Yanofsky, 1982) et sa concentration tend vers une valeur moyenne de 120 molécules par cellule d'*E. coli* K-12 en présence d'un excès de tryptophane exogène (Gunsalus, Gunsalus Miguel & Gunsalus, 1986). Cette concentration, si elle est suffisante pour réguler correctement l'activité de l'unique promoteur chromosomique Ptrp, peut s'avérer limitante face à plusieurs dizaines de vecteurs contenant le même promoteur. Quant au tryptophane, il peut aussi être un facteur limitant même s'il est apporté en excès dans le milieu de culture. Il existe en effet chez E. *coli* une activité tryptophanase codée par le gène *tna*A et capable de dégrader le tryptophane en indole, le détournant ainsi de sa fonction régulatrice (Snell, 1975). De plus, la tryptophanase est inductible par le tryptophane, ce qui rend vaine toute tentative de compenser ce phénomène de dégradation par une augmentation de l'apport en tryptophane.

Différentes approches visant à contrôler au mieux la fuite d'expression ont été envisagées et décrites. Cependant, certaines ont l'inconvénient d'être seulement applicables à l'échelle du laboratoire (Hasan & Szybalski, 1995 ; Suter-Crazzolara & Unsicker, 1995) ou de diminuer le rendement en produit recombinant (Stark, 1987).

Par conséquent, il existe aujourd'hui un grand besoin à développer un système d'expression de protéines recombinantes d'intérêt contrôlée et utilisable à grande échelle et permettant en particulier de contrôler la fuite d'expression. Ceci est justement l'objet de la présente invention.

L'invention concerne de nouvelles constructions basées sur le système d'expression Ptrp qui lorsqu'elles sont introduites dans une cellule hôte procaryote, de préférence de type bactérien, permettent de réduire l'expression résiduelle de gènes recombinants en début de culture, ces nouvelles constructions apportant un contrôle amélioré de la synthèse de protéines recombinantes.

La présente invention a pour objet une construction pour l'expression d'un gène codant pour une protéine recombinante d'intérêt placé sous le contrôle du promoteur de l'opéron tryptophane Ptrp dans une cellule hôte procaryote, caractérisée en ce que la construction comprend une séquence nucléique capable de modifier le gène codant pour une tryptophanase TnaA lorsque ladite séquence nucléique est introduite dans ladite cellule hôte, le produit d'expression dudit gène modifié étant incapable de dégrader le tryptophane en indole, et d'une séquence d'un promoteur suivie en 3' d'une séquence nucléique codant pour une molécule de nature ribonucléotidique ou protéique agissant négativement sur le promoteur Ptrp ou son produit de transcription, ladite molecule de nature ribonucléotidique agissant négativement sur le promoteur Ptrp étant choisie parmi les séquences ribonucléotidiques préférées telles que définies par la suite et ladite molécule de nature protéique agissant négativement sur le promoteur Ptrp étant choisie parmi la séquence codant pour l'aporépresseur TrpR de l'opéron tryptophane de ladite cellule hôte aucun de ses fragments biologiquement actifs ayant conservé son activité répresseur.

Par protéine recombinante d'intérêt, on entend désigner toutes protéines, polypeptides ou peptides obtenus par recombinaison génétique, et susceptibles d'être utilisés dans des domaines tels que celui de la santé humaine ou animal, de la cosmétologie, de la nutrition humaine ou animale, de l'agro-industrie ou de l'industrie chimique. Parmi ces protéines d'intérêt on peut citer en particulier mais sans s'y limiter :
- une cytokine et notamment une interleukine, un interféron, un facteur de nécrose tissulaire et un facteur de croissance et notamment liématopoïétique (G-CSF, GM-CSF), une hormone telle que l'hormone de croissance humaine ou l'insuline, un neuropeptide.
- un facteur ou cofacteur impliqué dans la coagulation et notamment le facteur VIII. le facteur von Willebrand, l'antithrombine III, la protéine C, la thrombine et l'hirudine.
- une enzyme et notamment la trypsine, une ribonucléase et la β-galactosidase,
- un inhibiteur d'enzyme telle que l'αl anti-trypsine et les inhibiteurs de protéases virales,
- une protéine capable d'inhiber l'initiation ou la progression de cancers, tels que les produits d'expression de gènes suppresseurs de tumeurs, par exemple le gène P53.
- une protéine capable de stimuler une réponse immunitaire ou un anticorps, telle que par exemple les protéines, ou leurs fragments actifs, de la membrane externe de bactérie Gram négatif, en particulier les protéines OmpA de Klebsiella ou la protéine G du virus respiratoire syncytial humain,
- une protéine capable d'inhiber une infection virale ou son développement, par exemple les épitopcs antigéniques du virus en cause ou des variants altérés de protéines virales susceptibles d'entrer en compétition avec les protéines virales nativcs,
- une protéine susceptible d'être contenue dans une composition cosmétique telle que la substance P ou une superoxydc dismutase,
- une protéine alimentaire,
- une enzyme capable de diriger la synthèse de composés chimiques ou biologiques, ou capable de dégrader certains composés chimiques toxiques, ou encore
- toute protéine ayant une toxicité vis-à-vis du micro-organisme qui la produit, en particulier si ce micro-organisme est la bactérie E. *coli*, comme par exemple, mais sans s'y limiter, la protéase du virus VIH-1, la protéine ECP (ECP pour "Eosinophile Cationic Protein") ou les protéines 2B et 3B du poliovirus.

Par séquence nucléique capable d'inactiver le gène codant pour une tryptophanase TnaA lorsque ladite séquence nucléique est introduite dans ladite cellule hôte, on entend désigner une séquence nucléique capable de modifier ledit gène de telle sorte que cette modification entraîne la perte de l'activité tryptophanase de ladite cellule hôte, le produit d'expression dudit gène modifié étant incapable de dégrader le tryptophane en indole, le détournant ainsi de sa fonction régulatrice. Parmi lesdites séquences nucléiques capables d'inactiver le gène codant pour une tryptophanase TnaA lorsqu'une desdites séquences nucléiques est introduite dans ladite cellule hôte, on préfère une séquence nucléique codant pour une tryptophanase TnaA inactivée obtenue par mutation telle que par substitution, insertion et/ou délétion d'au moins un nucléotide de la séquence nucléique codant pour une tryptophanase TnaA active.

L'invention comprend une construction selon l'invention, caractérisée en ce que la cellule hôte procaryote est une bactérie Gram négative, de préférence appartenant à l'espèce E. *coli*.

L'invention concerne également une construction selon l'invention, caractérisée en ce qu'elle comprend en outre en amont de ladite séquence nucléique capable d'inactiver le gène codant pour une tryptophanase TnaA lorsque ladite séquence nucléique est introduite dans ladite cellule hôte, tout ou partie de la séquence nucléique du promoteur Ptna de l'opéron tryptophanase.

De préférence, l'invention est relative à une construction selon l'invention, caractérisée en ce que ladite séquence nucléique capable d'inactiver le gène codant pour une tryptophanase TnaA lorsque ladite séquence nucléique est introduite dans ladite cellule hôte comprend un fragment muté de la séquence codante de ladite tryptophanase TnaA.

De manière préférée, l'invention est relative à une construction selon l'invention, caractérisée en ce que ledit fragment muté est obtenu par l'insenion d'un codon stop à une position telle que la séquence du fragment muté ainsi obtenu code pour un fragment protéique dépourvu d'activité tryptophanase.

De manière tout aussi préférée, l'invention est relative à une construction selon l'invention, caractérisée en ce que ledit fragment muté est un fragment muté de la séquence codante de la tryptophanase TnaA de ladite cellule hôte.

Pour ce qui concerne la séquence nucléique codant pour la tryptophanase TnaA de E. *coli*, et à son promoteur Ptna, on se référera dans la présente description à la séquence publiée par Deeley et Yanofsky (1981).

Pour ce qui concerne les séquences nucléiques codant pour le promoteur/opérateur Ptrp de l'opéron tryptophane. on se référera à la séquence publiée , par Yanofsky et al. (1981).

L'invention concerne également une construction selon l'invention dans laquelle ledit promoteur suivi en 3' d'une séquence nucléique codant pour une molécule de nature ribonucléotidique ou protéique agissant négativement sur le promoteur Ptrp est tout ou partie du promoteur Ptna de l'opéron tryptophanase d'E. *coli*.

De manière également préférée, l'invention comprend une construction selon l'invention, caractérisée en ce que ladite séquence nucléique codant pour une molécule de nature ribonucléotidique ou protéique agissant négativement sur le promoteur Ptrp, est la séquence codant pour l'aporépresseur TrpR de l'opéron tryptophane d'E. *coli* ou un de ses fragments biologiquement actifs telle que celle décrite par Gunsalus et Yanofsky (1980).

On entend désigner par une séquence nucléique comprenant tout ou partie de la séquence d'un promoteur, une séquence nucléique comprenant toute la séquence d'un promoteur, ou un de ses fragments biologiquement actifs, capable de diriger ou de contrôler l'expression d'un gène qui lui est relié de manière fonctionnelle.

On entendra désigner dans la présente description par fragment biologiquement actif d'un promoteur, toute séquence d'un fragment dudit promoteur, lequel fragment est capable de diriger ou de contrôler l'expression du gène situé en aval dudit fragment, ledit gène étant relié de manière fonctionnelle audit fragment.

On entendra désigner dans la présente description par fragment biologiquement actif de l'aporépresseur TrpR de l'opéron tryptophane, tout fragment dudit aporépresseur ayant conservé son activité répresseur.

Par séquence nucléique codant pour une molécule de nature ribonucléotidique agissant négativement sur le promoteur Ptrp, on préfère les ribonucléotidiques choisis parmi les séquences suivantes :
a) 5' - AUUCGCGUCU ACGGCUUCAU CGUGUUGCGC - 3'
b) 
5' - AUUCGCGUCU ACGGCUUCAU CGUGUUGCGC AGCACAACGC
GCCUGUCACC GGAUGUGUUU UCCGGUCUGA UGAGUCCGUG
AGGACGAAAC AGG - 3'
c) 
5' - AUUCAGUACG AAAAUUGCUU UCAUAAUUCU AGAUACCCUU
UUUACGUGAA CUU - 3'
d) 
5' - AUUCAGUACG AAAAUUGCUU UCAUAAUUCU AGAUACCCUU
UUUACGUGAA CUUAGCACAA CGCGCCUGUC ACCGGAUGUG
UUUUCCGGUC UGAUGAGUCC GUGAGGACGA AACAGG - 3'
e) 
5' - AUUCGCGUCU ACGGCUUCAU CGUGUUGCGC AUUCAGUACG
AAAAUUGCUU UCAUAAUUCU AGAUACCCUU UUUACGUGAA CUU - 3'
f) 
5' - AUUCGCGUCU ACGGCUUCAU CGUGUUGCGC AUUCAGUACG
AAAAUUGCUU UCAUAAUUCU AGAUACCCUU UUUACGUGAA
CUUAGCACAA CGCGCCUGUC ACCGGAUGUG UUUUCCGGUC
UGAUGAGUCC GUGAGGACGA AACAGG - 3'
g) 5' - CUUCGCGUCC UGAUGAGUCC GUGAGGACGA AACGGCUUCC - 3'
h)

5' - CUUCGCGUCC UGAUGAGUCC GUGAGGACGA AACGGCUUCC
AGCACAACGC GCCUGUCACC GGAUGUGUUU UCCGGUCUGA
UGAGUCCGUG AGGACGAAAC AGG - 3'.

Un autre aspect de l'invention concerne un vecteur contenant une construction selon l'invention.

De manière préférée, le vecteur selon l'invention est caractérisé en ce qu'il s'agit du vecteur pMAK705[tnaAt] ou du vecteur pMAK705[Ptna:trpR::3'tna].

L'invention concerne également une cellule hôte procaryote, de préférence une bactérie de l'espèce E. *coli*, transformée par un vecteur selon l'invention.

L'invention décrit un procédé de production de protéine recombinante dans une cellule hôte utilisant une construction selon l'invention.

Sous un autre aspect, l'invention a pour objet un procédé de production de protéines recombinantes d'intérêt, caractérisé en ce qu'il comprend les étapes suivantes :
a) la transformation d'une cellule procaryote par un vecteur selon l'invention, et l'intégration de ladite construction dans l'ADN de ladite cellule hôte ;
b) la transformation de ladite cellule procaryote par un vecteur contenant un gène codant pour ladite protéine recombinante d'intérêt ;
c) la culture de ladite cellule transformée dans un milieu de culture permettant l'expression de la protéine recombinante ; et
d) la récupération de la protéine recombinante a partir du milieu de culture ou de ladite cellule transformée.

L'invention a également pour objet un procédé de production d'une protéine recombinante d'intérêt selon l'invention, dans lequel ladite construction est introduite dans l'ADN de la cellule hôte procaryote.

L'invention décrit un procédé de production de protéines recombinantes selon l'invention, dans lequel ladite construction est introduite dans l'ADN de la cellule hôte procaryote par un vecteur selon l'invention, de préférence selon la méthode d'intégration chromosomique décrite dans l'exemple 1 ou 2.

L'invention décrit également un procédé de production de protéines recombinantes selon l'invention, dans lequel ladite construction est introduite sans autre élément d'ADN qui permettrait d'y associer un avantage sélectif.

L'invention comprend aussi un procédé de production d'une protéine recombinante d'intérêt selon l'invention, dans lequel ladite construction est introduite au locus de l'opéron tryptophanase.

L'invention a en outre pour objet un procédé de production de protéines recombinantes d'intérêt selon l'invention, caractérisé en ce que ledit procédé comprend en outre entre l'étape a) et b) du procédé ci-dessus, une étape de résolution et de criblage.

L'invention décrit en outre à un procédé de production de protéines recombinantes d'intérêt selon l'invention, dans lequel le contrôle de l'expression des protéines recombinantes avant induction du promoteur Ptrp est obtenu par l'induction dudit promoteur suivi en 3' d'une séquence nucléique codant pour une molécule de nature ribonucléotidique ou protéique agissant négativement sur le promoteur Ptrp selon l'invention.

Enfin, l'invention cite également un procédé de production selon l'invention, dans lequel l'induction dudit promoteur suivi en 3' d'une séquence nucléique codant pour une molécule de nature ribonucléotidique ou protéique agissant négativement sur le promoteur Ptrp selon l'invention est obtenue par tout moyen permettant d'exercer un effet inhibiteur ou activateur sur ledit promoteur.

De préférence, l'invention comprend un procédé de production selon l'invention, dans lequel l'induction dudit promoteur suivi en 3' d'une séquence nucléique codant pour une molécule de nature ribonucléotidique ou protéique agissant négativement sur le promoteur Ptrp selon l'invention est obtenue soit :
a) par le choix d'une source de carbone appropriée dans le milieu de culture ; soit
b) par l'ajout de tryptophane dans le milieu de culture ; ou par une combinaison de a) et b).

Les systèmes de constructions, de vecteurs, les cellules hôtes procaryotes transformées par lesdits vecteurs ainsi que les procédés de l'invention décrits ci-dessus et qui seront exemplifiés dans les exemples ci-après ici entrent dans le cadre du contrôle de la production de protéines recombinantes dans des cellules procaryotes. Ils sont adaptés à l'expression de gènes homologues ou hétérologues placés en aval du promoteur/opérateur Ptrp. Deux mutants sont plus particulièrement décrits ci-après pour illustrer l'invention. Ils portent les noms ICONE 100 et ICONE 200 (ICONE pour Improved Cells for Ovcr- and Non-leaky Expression). Les modifications introduites dans la lignée ICONE présentent les caractéristiques suivantes :
1) elles sont intégrées dans le chromosome de l'hôte,
2) étant générées par une technique de mutagenèse dirigée, elles sont ciblées à un endroit unique de l'ADN bactérien, parfaitement connu puisqu'il s'agit de l'opéron tna situé à 83 min sur la carte physique du génome d'E. *coli* K-12. En cela, les conséquences sur le plan physiologique pour l'hôte sont parfaitement identifiées. En particulier, la possibilité que des fonctions cryptiques soient réactivées suite à l'intégration chromosomique - comme cela est suspecté dans le cas de la mutagenèse aléatoire - est écartée,
3) la technologie employée dans ces exemples pour l'intégration chromosomique (Hamilton et al. (1989)) exclut la possibilité que d'autres séquences s'insèrent dans l'ADN bactérien. Notamment, les mutants ne portent pas de gène de résistance à un antibiotique. Dans le cas où ils seraient utilisés à l'échelle industrielle, ils offrent au producteur et au législateur la garantie qu'ils n'auront pas d'avantage sélectif en cas de dissémination accidentelle dans l'environnement.

Selon un aspect de l'invention, un premier type de mutant ou cellule transformée nommé ICONE 100 est décrit, qui porte une mutation dans le gène *tnaA* entraînant une perte de l'activité tryptophanase. Le phénotype associé à cette mutation est l'absence de dégradation du tryptophane. Ce type de mutant, après transformation par un vecteur rapporteur et culture sur un milieu favorisant habituellement l'activité tryptophanase, s'avère supérieur à l'isolat dont il est issu en termes de contrôle de la répression par le tryptophane.

Selon un autre aspect de l'invention, un second type de mutant nommé ICONE 200 est décrit, qui porte une cassette d'expression du gène *trp*R sous le contrôle du promoteur de la tryptophanase Ptna, intégrée au locus du gène *tna*A. L'utilisation du locus *tna* comme cible pour l'intégration entraîne chez la bactérie hôte une perte de l'activité tryptophanase qui se traduit comme décrit précédemment par une incapacité à convertir le tryptophane en indole. Par ailleurs, la présence de la cassette Ptna::trpR dans le chromosome confère à ce nouveau gène *trp*R les caractéristiques de Ptna, c'est-à-dire une sensibilité à la répression catabolique (Isaacs, Chao, Yanofsky, & Saier, 1994 ; Botsford & DeMoss. 1971) et l'inductibilité par le tryptophane (Stewart & Yanofsky, 1985). Cette dernière propriété constitue une innovation dans laquelle le promoteur Ptrp plasmidique est contrôlé au niveau de la transcription par un promoteur chromosomique, Ptna, qui lui est antagoniste. De manière surprenante, après transformation par un vecteur d'expression et culture en fermenteur, ICONE 200 s'avère supérieur à l'isolat dont il est issu en termes de contrôle de la répression par le tryptophane.

Les bactéries présentant une des caractéristiques mentionnées ci-dessus sont utites pour la production maîtrisée de molécules recombinantes. Aussi, la présente invention a également pour objet l'utilisation desdites bactéries transformées dans un procédé de production de protéines recombinantes.

Dans les exemples ci-dessous, l'avantage apporté par les deux mutants est clairement démontré en utilisant comme protéine recombinante la β-galactosidase d'Escherichia *coli*.

Un autre aspect de l'invention décrit les caractéristiques des mutations introduites. Celles-ci sont parfaitement définies, contrôlées sur le plan génétique et biochimique, ciblées au locus *tna* d'E. *coli* et exemptes de marqueur de sélection.

Les micro-organismes mutants ou transformés de l'invention sont construits à partir de procaryotes, plus précisément de bactéries Gram-négatives appartenant à l'espèce Escherichia. *coli.* Les propriétés du promoteur de l'opéron tryptophanase d'E *coli* (inductible par le tryptophane, sensible à la répression catabolique) ont été utilisées pour diriger la synthèse transitoire d'un médiateur agissant négativement sur l'expression dirigée par Pirp. Cependant, il est connu que d'autres espèces bactériennes, en particulier celles qui colonisent le tractus intestinal des animaux, sont capables de synthétiser une tryptophanase inductible par le tryptophane (Snell. 1975). Par conséquent, d'autres souches que E, *coli* conviennent pour pratiquer les méthodes décrites et y produire des protéines recombinantes.

Les exemples et les figures qui suivent sont destinés à illustrer l'invention sans aucunement en limiter la portée.

### Légendes des figures:

**FIGURE 1** : Cinétiques de croissance des souches RV308, ICONE 100 et ICONE 200 x pVA-βgal.
   DO 580 nm correspond à la mesure de la densité optique mesurée par spectrophotométrie.
**FIGURE 2 :** Cinétiques d'activité β-gal des souches RV308, ICONE 100 et ICONE 200 x pVA-βgal.
   Les bactéries transformées par un vecteur contenant le gène de la β-galactosidase sous le contrôle du promoteur Ptrp sont cultivées en fermenteur. L'activité β-galactosidase est mesurée par incubation d'un extrait cellulaire en présence d'ONPG (substrat spécifique de la β-galactosidase).
**FIGURE 3 :** Comparaison des cinétiques de croissance des souches E. coli RV308 et ICONE 200 transformées par le vecteur pVA-polio2B.
**FIGURES 4A et 4B :** Immuno-blot sur les extraits intracellulaires des cultures de RV308 et ICONE 200 transformées par le vecteur pVA-polio2B.

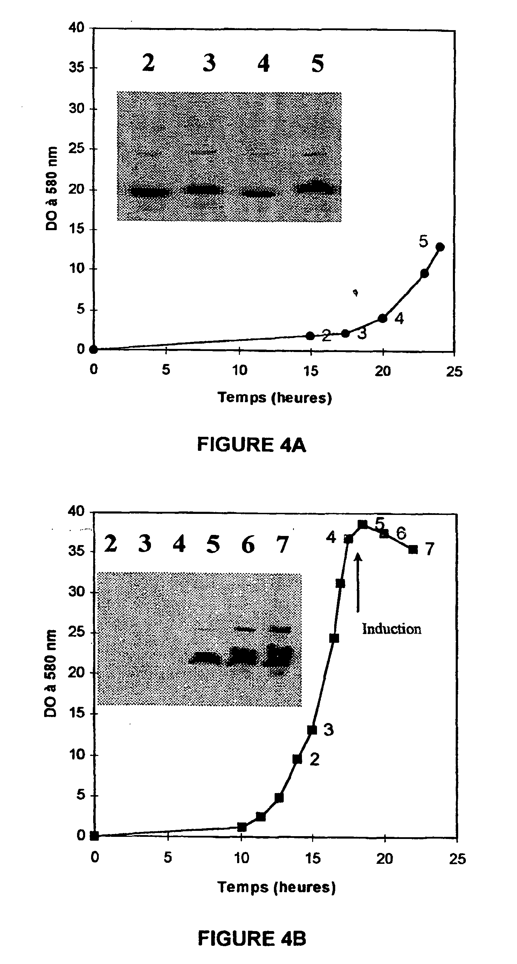

   Figure 4A : RV308 x pVA-polio2B
   Figure 4B : ICONE 200 x pVA-polio2B

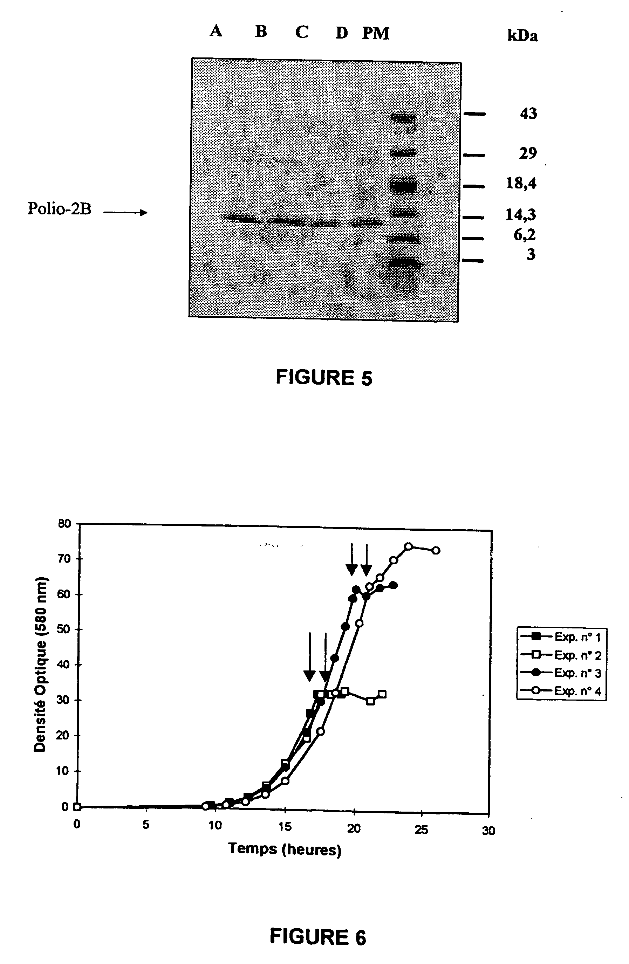

**FIGURE 5 :** Analyse par SDS-PAGE de la protéine polio-2B purifiée par chromatographie d'affinité sur Nickel.
   **A** : Expérience n° 2 : induction par 5 µg/ml d'IAA lorsque la densité optique est égale à 32,5 ;
   **B** : Expérience n° 1 : induction par 25 µg/ml d'IAA lorsque la densité optique est égale à 32,5 ;
   **C** : Expérience n° 4 : induction par 5 µg/ml d'IAA lorsque la densité optique est égale à 63,5 ;
   **D** : Expérience n° 3 : induction par 25 µg/ml d'IAA lorsque la densité optique est égale à 62,5 ;
   **PM** : Marqueur de poids moléculaire (kDa).
**FIGURE 6 :** Cinétiques de croissance de ICONE 200 x pVA-polio2B : influence du temps d'induction et de la concentration d'inducteur.
   ■ Expérience n° 1 : induction par 25 µg/ml d'IAA lorsque la densité optique est égale à 32,5.
   □ Expérience n° 2 : induction par 5 µg/ml d'IAA lorsque la densité optique est égale à 32,5.
   • Expérience n° 3 : induction par 25 µg/ml d'IAA lorsque la densité optique est égale à 62,5.
   ○ Expérience n° 4 : induction par 5 µg/ml d'IAA lorsque la densité optique est égale à 63,5.
Les flèches indiquent le moment de l'induction.

L'invention repose sur l'introduction stable de mutations dans le génome de la souche hôte. Toutes les modifications présentées dans les exemples ci-après sont introduites au locus *tna* d'E. *coli*, constitué schématiquement de l'enchaînement suivant :
A) promoteur Ptna,
B) séquence codante du gène tnaA (tryptophanase),
C) région intergénique,
D) séquence codante du gène tnaB (tryptophane-perméase),
E) terminateur de transcription.

Plus précisément, les modifications portent sur l'élément (B). Celui-ci est remplacé au profit d'un élément (b) dont les caractéristiques dans les diverses constructions sont les suivantes :

**Tableau 1 : Nature des mutations portées par ICONE 100 et ICONE 200**

| Nom du mutant | Nature de l'élément (b) |
|---|---|
| ICONE 100 | séquence codante de tnaA interrompue en position + 221 par un codon stop et un site de restriction Xbal |
| ICONE 200 | séquence codante du gène trpR codant pour l'aporépresseur de Ptrp |

### Exemple 1 : Construction du mutant ICONE 100

Un fragment d'ADN noté tnaAT est amplifié par PCR. 11 s'étend des positions - 275 à + 1054 par rapport au premier nucléotide de la séquence codante de *tnaA*. Ce fragment qui chevauche le promoteur Ptna et tnaA est amplifié par réaction PCR en deux parties. La partie 1 s'étend de la position - 275 à la position + 220. Elle est amplifiée à l'aide des oligonucléotides Trp5 (sens) et Trp2 (anti-sens) dont la séquence est :

Trp5 : 5' - CGGGATCCGTGTGACCTCAAAATGGTT - 3'
BamHI

Trp2 : 3' – CTACGCGCCGCTGCTTCGGATTAGATCTCG - 5'
(anti-sens)                                        stop  XbaI

La partie II se situe dans la séquence codante de tnaA, immédiatement en 3' de la partie I. Elle s'étend des positions + 221 à + 1054. Elle est amplifiée à l'aide des oligonucléotides Trp3 et Trp4 :

Trp3 : 5' – CGTCTAGACAGCGGCAGTCGTAGCTAC - 3'
XbaI

Trp4 : 3' – CCTTCTCTAACCGCAACAGTTCGAACG - 5'
(anti-sens) HindIII

Les réactions de PCR sont effectuées en utilisant comme matrice des colonies d'*E*. *coli* K-12 lysées dans le tampon de la Taq polymérase (AmpliTaq Gold CETUS, USA).

Les produits d'amplification sont précipités à l'éthanol puis digérés avec les enzymes de restriction appropriées (BamHI - XbaI pour la partie I, XbaI - HindIII pour la partie II). Une analyse sur gel d'agarose coloré au BET permet de vérifier que les fragments ont la taille attendue (Deeley & Yanofsky, 1981). Le fragment tnaAT est généré en liguant les deux fragments I et II au site XbaI. Il diffère de la séquence naturelle par la présence d'un codon stop en position + 221 suivi d'un site de restriction XbaI. Ce fragment tnaAT est cloné aux sites BamHI / HindIII dans le vecteur pRIT28 (Hultman, StahI, Hornes & Uhlen, 1989) et séquencé. Le fragment tnaAT est sous-cloné dans le vecteur pMAK705 (Hamilton, Aldea, Washburn, Babitzke & Kushner, 1989), donnant pMAK705[tnaAT].

La méthode employée pour générer un réarrangement génétique chez *E. coli* est celle décrite par Hamilton et al. (1989). Elle est basée sur l'emploi du vecteur suicide pMAK705 qui porte une origine de réplication thermosensible, fonctionnelle à 30°C mais inactive au-delà de 42°C, ainsi que le gène de résistance au chloramphénicol (CMP). *E. coli* RV308 (Maurer, Meyer & Ptashne, 1980) est transformé par 4 µg du vecteur pMAK705[tnaAT] et le mélange de transformation est étalé sur boîtes de milieu LB + CMP 20 µg/ml. Après incubation une nuit à 30°C, trois clones sont repiqués en milieu liquide LB + CMP 20 µg/ml et incubés à 30°C sous agitation jusqu'à atteindre une DO à 580 nm voisine de 1. Les suspensions sont ensuite étalées sur milieu LB + CMP 20 µg/ml et incubées à 44°C et à 30°C. Les colonies qui se développent à 44°C (= co-intégrants) sont porteuses d'une intégration chromosomique du vecteur, cette intégration étant favorisée par l'existence d'homologies de séquence entre le chromosome et l'insert porté par le vecteur.

La phase dite de résolution consiste à favoriser l'excision du vecteur par un mécanisme de recombinaison entre des séquences répétées présentes sur le chromosome. Quelques clones isolés à 44°C sont cultivés en milieu liquide LB + CMP 20 µg/ml à 30°C pendant trois jours en renouvelant le milieu régulièrement. Les suspensions sont ensuite diluées, étalées sur milieu gélosé LB + CMP 20 µg/ml et incubées à 30°C jusqu'à l'apparition de colonies individualisées. Plusieurs dizaines de colonies sont repiquées en double sur milieu gélosé LB + CMP 20 µg/ml à 30°C et 44°C. Les colonies qui ne se développent pas à 44°C sont retenues et criblées par une réaction de PCR indiquant si la résolution du vecteur a conservé le codon stop et le site Xbal au locus *tna*. Les oligonucléotides utilisés sont Trp6 (sens) et Trp7 (anti-sens), respectivement homologues à la mutation désirée et à une partie du terminateur de tnaA :

Trp6 : 5' - CGACGAAGCCTAATCTAGA - 3'
stop XbaI

Trp7 : 3' – CCGATATTCCTACAATCGG - 5'
(anti-sens)

Sur dix-huit clones criblés, neuf donnent un fragment d'amplification de la taille attendue indiquant la présence du codon stop suivi du site XbaI dans le gène tnaA. Les neuf autres clones ne donnent pas de produit d'amplification, probablement parce que l'étape de résolution a restauré sur le chromosome le gène *tnaA* non muté. Parmi les neuf clones positifs, quatre sont prélevés et soumis à un curage du plasmide par repiquages successifs en absence de pression de sélection. Après quelques jours de culture, on obtient des clones redevenus sensibles au chloramphénicol.

La présence de la mutation inactivant *tnaA* est confirmée de deux manières différentes : d'abord, une amplification par PCR à l'aide des oligonucléotides Trp5 et Trp4 suivie d'une digestion par Xbal montre que le site de restriction, absent chez E. *coli* RV308, est présent dans le gène tnaA des mutants ; ensuite, par une culture des mutants dans un milieu riche en tryptophane suivie du test à l'indole (ajout du réactif de Kovacs dans le milieu de culture), on montre que les mutants n'ont pas généré d'indole alors que la souche RV308 d'origine produit de l'indole dans les mêmes conditions. On en déduit que la mutation introduite entraîne une perte de l'activité tryptophanase.

Un clone est sélectionné en vue d'une conservation sous forme congelée. Il est nommé ICONE 100.

### Exemple 2 : Construction du mutant ICONE 200

Un fragment d'ADN est construit in vitro par amplification PCR de trois sous-unités.

La première sous-unité située dans le promoteur Ptna s'étend des positions - 511 à + 3 par rapport au premier nucléotide de la séquence codante de *tnaA.* Elle est amplifiée à partir des oligonucléotides TrpR1 (biotinylé en 5') et TrpR2 :

TrpR1 :     5' – CTGGATCCCTGTCAGATGCGCTTCGC - 3'
BamHI

TrpR2 :        3' – CTTCCTAATACATTACCGGGTTG - 5'
(anti-sens)

La seconde sous-unité correspond à la séquence codante du gène *trpR* d'*E. coli.* Elle est amplifiée à partir des oligonucléotides TrpR3 et TrpR4 (biotinylé en 5') :

TrpR3 :          5' – GTAATGGCCCAACAATCACC - 3'
                              start

TrpR4 : 3' – CACAACGACTTTTCGCTAACTGACGTCAG - 5'
(anti-sens) PstI

La troisième sous-unité correspond à la séquence située immédiatement en 3' de la séquence codante de *tnaA*. Elle contient la région intergénique de l'opéron tna et une partie du gène *tnaB* codant pour la tryptophane-perméase. Elle est amplifiée à partir des oligonucléotides TrpR5 et TrpR6 :

TrpR5 :     5' – CGCTGCAGTTAATACTACAGAGTGG - 3'
PstI

TrpR6 :        3' – CCAGCTAATGAGGTAAGTTCGAAC - 5'
(anti-sens)                                        HindIII

Les fragments amplifiés sont puritiés selon la méthode GeticClean (Bio101, La Jolla, CA, USA).

Les sous-unités I et II sont fusionnées de la manière suivante. Dans deux tubes séparés, chaque sous-unité est mise en incubation avec 30 µl de billes marquées à la streptavidine (Dynabeads, DYNAL, Norvège). Après 20 min à 37°C et 5 min à température ambiante, l'ADN fixé est dénaturé par 50 µl de NaOH 0,15 M. Les ADN simple-brin récupérés dans chaque surnageant sont mélangés à parts égales et soumis à une réaction d'hybridation et d'extension en présence de la Taq polymérase (AmpliTaq Gold, CETUS, USA) suivant cinq cycles de PCR. Le produit de la réaction est amplifié dans une réaction PCR à l'aide des oligonucléotides TrpR1 et TrpR4.

Le produit d'amplification purifié par GeneClean est digéré par BamHI et PstI. Le fragment ainsi isolé est cloné dans le vecteur pRIT28 pour donner pRIT28[Ptna::trpR], puis séquencé.

La sous-unité III est digérée par les enzymes PstI et HindIII puis clonée dans pRIT28 pour donner pRIT28[3'tna], puis la séquence est vérifiée par séquençage ADN (ABI 373A, Perkin Elmer, USA).

Le vecteur pRIT28[Ptna::trpR] est digéré par les enzymes PstI et HindIII puis ligué en présence de la sous-unité III elle-même isolée à partir de pRIT28[3'tna] par double digestion PstI/HindIII. Le vecteur résultant est nommé pRIT28[Ptna::trpR::3'tna]. L'insert est transféré dans pMAK705 après double digestion par les enzymes BamHI et HindIII. Le plasmide résultant est nommé pMAK705[Ptna::trpR::3'tna].

L'intégration de la fusion Ptna::trpR::3'tna au locus tna d'E. *coli* RV308 est réalisée dans des conditions analogues à celles décrites dans l'exemple 1. Brièvement, la souche est transformée par le vecteur pMAK705[Ptna::trpR::3'tna] puis soumise aux étapes d'intégration et de résolution.

Le criblage des colonies en fin de résolution fait appel à des conditions légèrement différentes de celles de l'exemple 1. Le locus tna est amplifié par PCR à partir des oligonucléotides TrpR11 et TrpR7 :

TrpR11 : 5' – GGGCAGGTGAACTGCTGGCG - 3'

TrpR7 :        3' - GGTGCCGTTATAAGGGTCGGAC - 5'
(anti-sens)

TrpR1 s'hybride avec la séquence de Ptna en amont (5') de TrpR1, et TrpR7 s'hybride avec la séquence de *tnaB*, en aval (3') de TrpR6. Le produit d'amplification a une taille différente suivant que le gène placé en aval de Ptna est *tnaA* (situation rencontrée dans RV308) ou *trpR* (situation recherchée chez les mutants). Une colonie possédant *trpR* au locus *tna* est conservée et nommée ICONE 200. Une analyse de sa séquence chromosomique montre qu'elle possède le gène *trpR* immédiatement en aval du promoteur Ptna. Une culture en présence de tryptophane atteste l'absence de formation d'indole, ce qui est une conséquence logique de la perte du gène *tnaA*.

### Exemple 3 : Fuite d'expression en présence de succinate + tryptophane

Cet exemple décrit les capacités relatives de E. *coli* RV308, ICONE 100 et ICONE 200 à contrôler l'expression d'une protéine recombinante sous le contrôle du promoteur Ptrp. A cet effet, nous avons construit un vecteur d'expression noté pVA-βgal dans lequel la séquence codant pour la β-galactosidase d'E. *coli* est placée en aval du promoteur Ptrp. Le vecteur d'origine utilisé pour cette construction est pVAABP308 (Murby, Samuelsson, Nguyen, et al., 1995).

Chacune des trois souches est transformée par le vecteur pVA-βgal. Les transformants obtenus sont cultivés individuellement dans un milieu complet (Tryptic Soy Broth (DIFCO) 30 g/l, Yeast Extract (DIFCO) 5 g/l) pendant une nuit à 37°C. Un aliquot de ces précultures est transféré dans 60 ml de milieu M9.YE.SUCC (solution de sels M9 IX (DIFCO), Yeast Extract (DIFCO) 5 g/l, succinate de sodium 20 g/l). Après un temps d'incubation à 37°C permettant d'atteindre la phase exponentielle de la croissance, un prélèvement est effectué sur chaque culture. La croissance est estimée par la Densité Optique à 580 nm de la suspension bactérienne. Le niveau d'activité β-galactosidase est mesuré dans chaque culot cellulaire. Pour cela, 1 ml de culture est centrifugé 3 min à 12 000 g. Le culot cellulaire est repris dans 900 µl de tampon (Tris-HCl 50 mM pH 7,5 - EDTA 1 mM - NaCl 100 mM - lysozyme 400 µg/ml) et incubé 15 min à 37°C. 100 µl de SDS (1 % dans Tris-HCl 50 mM pH 7,5) sont ajoutés et l'échantillon est placé 5 min à température ambiante. Le dosage s'effectue en mélangeant 30 µl d'échantillon, 204 µl de tampon (Tris-HCl 50 mM pH 7,5 - MgCl₂ 1 mM) et 66 µl d'ONPG (4 mg/ml dans Tris-HCl 50 mM pH 7,5). Le mélange réactionnel est placé en incubation à 37°C. La réaction est stoppée par l'ajout de 500 µl de Na₂CO₃ 1 M. La DO à 420 nm rapportée au temps d'incubation est proportionnelle à l'activité β-galactosidase présente dans l'échantillon. Sachant que E. *coli* RV308 a une délétion complète de l'opéron lac (Maurer, Meyer & Ptashne, 1980), l'activité β-galactosidase mesurée est uniquement due à l'expression du gène porté par le vecteur pVA-βgal.

Le tableau 2 résume les résultats obtenus avec chacune des souches RV308, ICONE 100 et ICONE 200.

**Tableau 2 : Croissance des souches RV308, ICONE 100 et ICONE 200 et fuite d'expression (moyenne et écart-type sur trois expériences)**

| | DO 580 nm | β-GAL (U/ml) |
|---|---|---|
| RV308 | 2,47 ± 0,01 | 0,93 ± 0,09 |
| ICONE 100 | 3,69 ± 0,24 | 0,21 ± 0,03 |
| ICONE 200 | 2,43 ± 0,03 | 0,02 ± 0,00 |

Les résultats du tableau 2 montrent que les mutants de la lignée ICONE se développent au moins aussi bien que la souche RV308 dont ils sont issus. Les mutations introduites n'ont donc pas d'effet négatif sur la croissance. Par ailleurs, l'activité β-galactosidase mesurée est différente chez les trois souches. ICONE 100 possède une activité intracellulaire environ 4,5 fois inférieure à celle de RV308. Dans des conditions - succinate comme source de carbone - où l'activité du promoteur Ptna est maximale (Botsford & DeMoss, 1971), la délétion du gène de la tryptophanase conduit donc à une réduction de la fuite d'expression, probablement en limitant la dégradation du tryptophane intracellulaire (co-répresseur). Dans ces mêmes conditions, le niveau de fuite d'expression chez ICONE 200 est encore diminué d'un facteur 10 par rapport à ICONE 100. L'activité du promoteur Ptrp plasmidique est donc minimale chez ICONE 200. D'une part, la perte de l'activité tryptophanase donne à la bactérie la possibilité de mieux contrôler Ptrp comme cela est démontré pour ICONE 100. Mais ICONE 200 possède une seconde caractéristique qui la distingue d'ICONE 100 sur le plan génétique et lui donne au niveau expérimental un avantage supplémentaire en termes de contrôle de l'expression. Ainsi, dans des conditions où Ptna est actif, ICONE 200 a la possibilité de diriger la surexpression de l'aporépresseur TprR et par conséquent la fuite d'expression mesurée au niveau du promoteur Ptrp plasmidique est réduite d'un facteur proche de 50 par rapport à la souche d'origine RV308.

### Exemple 4 : Fuite d'expression en présence de glycérol + tryptophane

Cet exemple démontre l'avantage apporté par le mutant ICONE 200 dans un milieu de culture et des conditions de fermentation proches de celles qui pourraient être utilisées industriellement pour produire des protéines recombinantes avec le système Ptrp.

Chacune des trois souches RV308, ICONE 100 et ICONE 200 est transformée par le vecteur pVA-βgal. Les transformants obtenus sont cultivés individuellement dans 200 ml de milieu complet (Tryptic Soy Broth (DIFCO) 30 g/l, Yeast Extract (DIFCO) 5 g/l) pendant une nuit à 37°C.

La suspension cellulaire obtenue est transférée stérilement dans un fermenteur (modèle CF3000 de Chemap, capacité 3,5 1) contenant 1,8 litres du milieu suivant (concentrations pour 2 litres de culture finale) : glycérol 90 g/l, (NH₄)2SO₄ 5 g/l, KH₂PO₄ 6 g/l, K₂HPO₄ 4 g/l, Na3-citrate 2H₂O 9 g/l, MgSO₄ 7H₂O 2 g/l, extrait de levure 1 g/l, CaCl₂ 2H₂O 30 mg/l, ZnSO₄ 7H₂O 8 mg/l, CoCl₂ 6H₂O 7 mg/l, Na₂MoO₄ 2H₂O 7 mg/l, MnSO₄ 1H₂O 10 mg/l, H₃BO₃ 2 mg/l, CuSO₄ 5H₂O 8 mg/l, FeCl₃ 6H₂O 54 mg/l, antimousse 0,06 %, tétracycline 8 mg/l, tryptophane 200 mg/l. Le pH est régulé à 7,0 par ajout d'ammoniaque. Le taux d'oxygène dissous est maintenu à 30 % de la saturation par asservissement de la vitesse d'agitation puis du débit d'aération à la mesure de l'O2 dissous. Lorsque la Densité Optique de la culture atteint une valeur comprise entre 40 et 45, on procède à l'induction par l'ajout de 25 mg/l d'acide indole-acrylique (SIGMA).

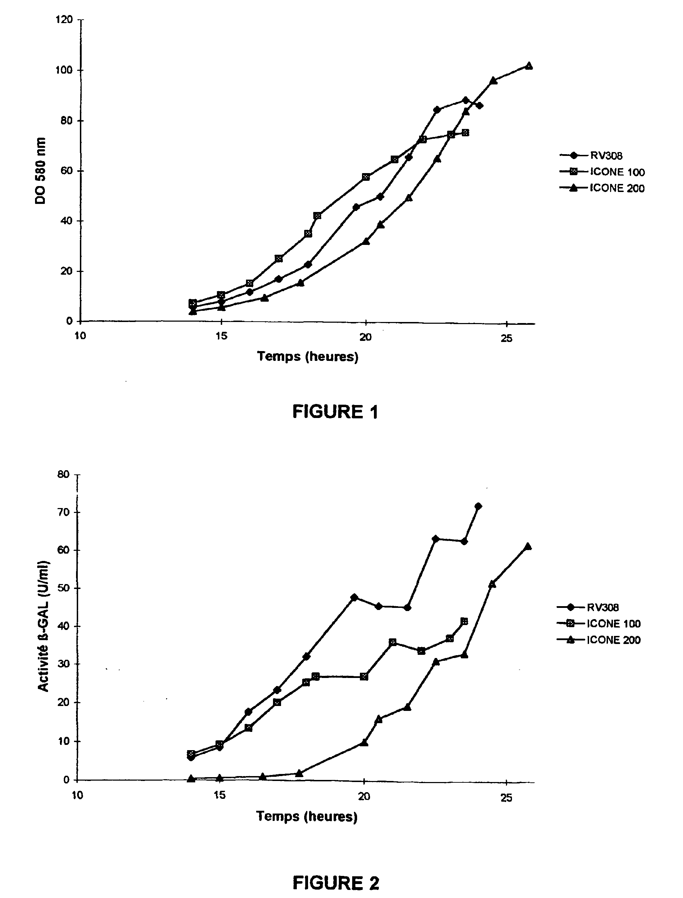

Une analyse en cinétique de la densité optique de la culture (DO à 580 nm de la suspension) et de l'activité β-galactosidase intracellulaire (voir exemple 3) est effectuée. Les figures 1 et 2 illustrent respectivement les cinétiques de croissance et d'activité β-galactosidase des trois cultures.

Les données présentées en figure 1 confirment l'observation de l'exemple 3 : les trois souches ont des cinétiques de croissance comparables. Les mutants de la lignée ICONE ont de ce point de vue conservé le potentiel de croissance de E. *coli* RV308 et ils demeurent donc des candidats potentiels pour une utilisation industrielle.

Les données de la figure 2 montrent l'impact des mutations portées par les souches ICONE sur l'expression de la β-galactosidase en fermenteur. Clairement, sur un milieu à base de glycérol, la présence ou l'absence de l'activité tryptophanase n'a pas d'effet sur le contrôle de l'expression comme en atteste la première partie des courbes RV308 et ICONE 100, même si l'on constate que le tryptophane exogène disparaît plus rapidement dans la culture RV308 que dans celle d'ICONE 100 (données non présentées). En revanche, le mutant ICONE 200 présente de meilleures capacités à contrôler l'expression en début de culture : l'activité β-galactosidase reste faible pendant les 18 premières heures de culture puis commence à augmenter à partir de t = 20 h, moment où la concentration en tryptophane extracellulaire devient nulle (données non présentées). La deuxième partie de la courbe concernant ICONE 200 montre que l'activité β-galactosidase augmente régulièrement pour atteindre un niveau en fin de culture proche de celui obtenu avec RV308. En cela, nous démontrons que le système de régulation présent chez ICONE 200 apporte un contrôle transitoire du promoteur Ptrp plasmidique. Ce contrôle, exercé par le tryptophane et/ou la source de carbone, devient inopérant dans la deuxième partie de la culture et ne vient pas s'opposer à une expression maximale de la protéine recombinante.

### Exemple 5 : Contrôle de l'expression d'une protéine toxique

Cet exemple décrit le comportement des souches RV308 et ICONE 200 en culture lorsqu'elles sont transformées par un vecteur portant, en aval du promoteur tryptophane, le gène d'une protéine toxique. A titre d'exemple et pour illustrer l'invention, le gène d'intérêt est celui codant pour la protéine 2B du poliovirus. Il a été décrit que la surexpression de cette protéine modifie la perméabilité des membranes chez les bactéries (Lama et al., 1992) ainsi que dans les cellules eucaryotes (Aldabe et al., 1996), ce qui en fait un modèle de choix pour étudier les conséquences de la fuite d'expression chez E. *coli*.

Le gène codant pour la protéine 2B est amplifié à partir du vecteur pET3.2B (Lama et al., 1992) par une réaction de PCR à l'aide des oligonucléotidcs suivants : -

PO2.1 5' – GCGAATTCTGGCATCACCAATTACATAG - 3'
(sens) EcoRI

PO2.21 5' - GCAAGCTTAGTGGTGGTGGTGGTGGTGTTGCTTGATGACATAA
(anti-sens) HindIII
GGTATC - 3'

Le produit d'amplification est ensuite digéré par les enzymes de restriction EcoRI et HindIII puis cloné dans un vecteur d'expression dérivé de pBR322 portant le promoteur tryptophane Ptrp. Le vecteur résultant, nommé pVA-polio2B, porte une séquence codant pour la protéine 2B fusionnée à son extrémité C-terminale à une queue poly(His), sous le contrôle du promoteur Ptrp.

Le vecteur pVA-polio2B est introduit dans les bactéries E. *coli* RV308 et ICONE 200 par transformation. Un clone recombinant de chaque construction est ensuite cultivé dans des conditions similaires à celles décrites dans l'exemple 4.

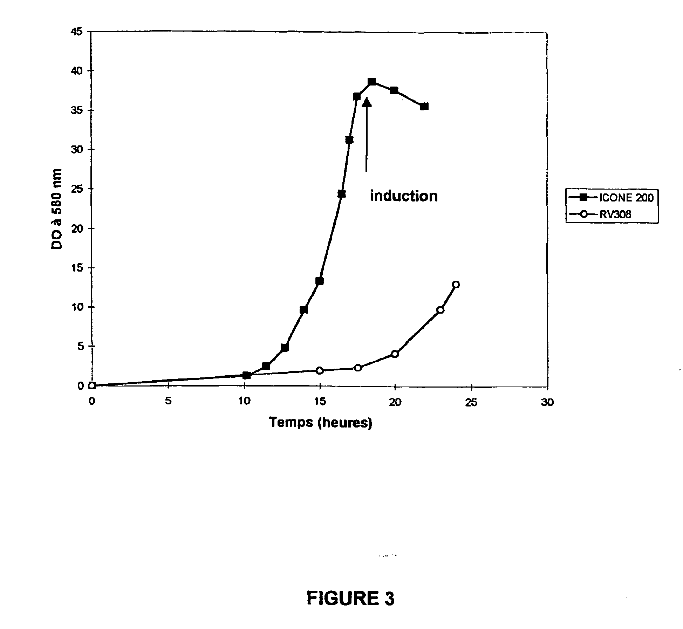

Les cinétiques de croissance des bactéries RV308 et ICONE 200 mesurées par la densité optique à 580 nm sont présentées en figure 3. Il y apparaît nettement que RV308 présente un retard de croissance important : le temps de génération moyen en fermenteur pendant les premières 14 heures de culture est de 1 h 45 min. contre seulement 1 h 17 min. pour ICONE 200. Après 24 heures de culture, la densité optique pour la souche RV308 est seulement égale à 13. De manière surprenante, la souche ICONE 200 atteint, elle, une densité optique égale à 37 après 17 h 30 min. de culture, âge où est effectuée l'induction par ajout d'acide indole acrylique (IAA) à 25 µg/ml. L'effet de l'induction est immédiat : la vitesse de consommation d'oxygène chute brusquement (données non présentées) et la croissance s'arrête.

Des prélèvements à différents temps de culture ont été effectués et analysés pour leur contenu en protéine recombinante. Des échantillons de biomasse centrifugés à 8 000 g sont repris par un tampon P1 (Tris 25 mM, EDTA 1,1 mM, lysozyme 1 mg/ml, pH 8) à raison de 5 ml pour 1 g de biomasse. La biomasse est remise en suspension, incubée 15 min. à température ambiante puis soumise à une sonication pendant 2 min. Le lysat est centrifugé de nouveau (10 000 g, 15 min., 4°C) pour donner une fraction soluble (surnageant) et une fraction insoluble (culot repris par 200 µl de tampon P2 : Tris 25 mM, EDTA 1 mM, pH 8). Ces échantillons sont déposés sur gel de polyacrylamide et soumis à une électrophorèse en conditions dénaturantes (SDS-PAGE). Le gel est ensuite transféré sur membrane selon la technique du Western-blot pour révéler la présence de la protéine recombinante. L'anticorps utilisé est un monoclonal anti-poly(His) couplé à la péroxidase (Sigma). La révélation est effectuée par chimioluminescence avec le kit ECL+ (Amersham). Les figures 4A et 4B présentent le résultat des immuno-blots sur les fractions insolubles issues respectivement des cultures de RV308 et ICONE 200. La figure 4A montre que la protéine recombinante est présente dans tous les prélèvements, c'est-à-dire dès le début de la culture jusqu'au temps de fermentation t = 24 h alors qu'aucune induction par l'IAA n'a été effectuée. En revanche, avec ICONE 200, aucune protéine recombinante n'est détectée avant induction (figure 4B). C'est seulement après induction par l'IAA que la protéine 2B est détectable (dans la fraction insoluble) et que la manifestation de son caractère toxique est observée. Ainsi, ces résultats mettent en évidence que le mutant ICONE 200 a un avantage manifeste par rapport à la souche RV308 dont il est issu et permet de réaliser un contrôle efficace de l'expression en fermenteur.

### Exemple 6 : Production d'une protéine toxique

Cet exemple vise à démontrer qu'une protéine toxique peut être exprimée dans une culture d'E. *coli* ICONE 200 à forte densité cellulaire dans des conditions de culture adaptées à une extrapolation industrielle. Dans ce but, la souche E. *coli* ICONE 200 transformée par le vecteur pVA-polio2B est évaluée. Les résultats obtenus à l'exemple 5 indique que les conditions d'induction doivent être optimisées si l'on veut éviter que l'expression se traduise instantanément par un arrêt de croissance puis par une lyse bactérienne. Aussi, cet exemple décrit différents essais destinés à optimiser le rendement en protéine recombinante par unité de volume fermenté en jouant sur la concentration d'inducteur et la densité cellulaire à l'induction. Les conditions de culture utilisées sont celles décrites dans l'exemple 4.

Les combinaisons expérimentales testées sont les suivantes :
■ Expérience n° 1 : induction par 25 µg/ml d'IAA lorsque la densité optique est comprise entre 30 et 35 ;
■ Expérience n° 2 : induction par 5 µg/ml d'IAA lorsque la densité optique est comprise entre 30 et 35 ;
■ Expérience n° 3 : induction par 25 µg/ml d'IAA lorsque la densité optique est comprise entre 60 et 65 ;
■ Expérience n° 4 : induction par 5 µg/ml d'IAA lorsque la densité optique est comprise entre 60 et 65.

Pour chaque expérience, des échantillons de biomasse sont prélevés à différents temps après l'induction et analysés selon le protocole suivant. Environ 20 grammes de biomasse sont repris dans 100 ml de Start Buffer 1X (préparé à partir du concentré 8X : 1,42 g Na₂HPO₄ 2H₂O, 1,11 g NaH₂PO₄ H₂O, 23,38 g NaCl, q.s.p. 100 ml, pH 7,4). La suspension est traitée par sonication 3 x 5 min puis centrifugée 30 min à 20 000 g, 4°C. Le culot est repris par 15 ml de Start Buffer + guanidine-HCl 6 M + 0,1 % SB3-14 (N-tétradécyl-N,N-diméthyl-3-ammonio-1-propanesulfonate, Sigma) puis mis en incubation dans la glace pendant 1 heure. La suspension est centrifugée 1 heure à 30 000 g, 4°C. Le surnageant est filtré sur 0,45 µ en vue de sa purification par chromatographie d'affinité sur métal chélaté. Une colonne contenant 1 ml de gel (HiTrap Chelating, Amersham Pharmacia Biotech) est chargée avec 1 ml de NiSO₄ 0,1 M, lavée par 5 ml d'eau puis équilibrée par 30 ml de Start Buffer + guanidine-HCl 6 M + 0,1 % SB3-14. L'échantillon est ensuite chargé sur la colonne. Un rinçage avec 60 ml de Wash Buffer (Start Buffer + guanidine-HCl 6 M + 0,1 % SB3-14 + imidazole 20 mM) permet d'éliminer la majorité des protéines fixées par des interactions non spécifiques. La protéine recombinante polio-2B est éluée par 10 x 1 ml de tampon d'élution (Start Buffer + guanidine-HCl 6 M + 0,1 % SB3-14 + imidazole 300 mM). Les fractions les plus concentrées en protéines sont regroupées puis dessalées sur gel Sephadex G-25 (colonnes PD10, Amersham Pharmacia Biotech). La qualité et la quantité de protéine polio-2B ainsi obtenue sont estimées respectivement par électrophorèse en conditions dénaturantes (SDS-PAGE) et par un dosage des protéines totales (méthode au BCA, Pierce).

La figure 5 présente un gel SDS coloré au bleu de Coomassie des protéines polio-2B extraites et purifiées à la suite des expériences 1 à 4 décrites ci-dessus. La taille de la protéine recombinante correspond à la taille théorique (11 kDa) prédite à partir de sa séquence codante. De plus, elle correspond à la taille de la protéine majoritaire observée en Wcstcrn-blot après induction, dans le lysat d'E. *coli* ICONE 200 x pVApolio-213 (figure 4B). Il est donc vraisemblable que les protéines visibles sur la figure 5 correspondent à la protéine 2B du poliovirus fusionnée à une qucuc polyhistidine. Par ailleurs, la qualité des protéines obtenues est identique dans toutes les conditions d'induction testées.

Le tableau 3 ci-après résume les résultats obtenus en combinant différents facteur tels que Densité Optique à l'induction, concentration en inducteur et temps de culture après induction.

**Tableau 3: Influence de la Densité Optique à l'induction, de la concentration en inducteur et du temps après induction sur le rendement d'expression d'une protéine toxique (exemple de polio-2B)**

| N° d'expérience | Densité Optique (580 nm) de la culture au moment de l'induction | Concentration en inducteur (IAA, mg/l) | Temps après induction (HH :MM) | Quantité de protéine extraite et purifiée (mg par litre de milieu) |
|---|---|---|---|---|
| 1 | 32,5 | 25 | 00 :45 | 3 |
| | | | 01 :45 | 2 |
| 2 | 32,5 | 5 | 00 :45 | 6 |
| | | | 01 :45 | 6 |
| 3 | 62,5 | 25 | 00 :45 | 5,5 |
| | | | 02 :45 | 7,5 |
| 4 | 63,5 | 5 | 00 :45 | 6 |
| | | | 02 :50 | 9 |

En comparant les groupes d'expériences (1-2) et (3-4), on observe que plus l'induction est réalisée tardivement, plus le rendement d'expression est élevé. Ceci confirme l'intérêt de faire croître la biomasse le plus possible avant de déclencher l'induction. Dans le cas des expériences (3-4), environ 70 % du substrat carboné est consommé au moment où l'expression de la protéine polio-2B est déclenchée. Avec une souche telle que ICONE 200, la phase de croissance cellulaire et la phase d'expression sont totalement séparées, ce qui permet d'optimiser le rendement en protéine recombinante, même lorsque cette protéine est toxique.

Parallèlement au temps d'induction, la concentration en inducteur est également un paramètre influent. Le meilleur résultat d'expression obtenu dans cet exemple correspond à l'expérience n° 4 où la concentration d'inducteur rapportée au nombre de cellules est la plus faible (5 mg/l dlAA pour une culture dont la densité optique est égale à 63,5). C'est également dans cette expérience que l'effet toxique de l'expression de polio-2B est le moins marqué puisque la culture continue à se développer après l'induction alors que la croissance est stoppée complètement dans toutes les autres expériences (voir figure 6). Il est donc particulièrement important d'ajuster les conditions d'induction d'une protéine toxique, de manière à trouver l'optimum entre une concentration en inducteur trop faible pour donner lieu à une expression significative et une concentration trop forte provoquant l'arrêt immédiat du métabolisme bactérien. En comparant les résultats des expériences n° 4 et n° 1, on observe qu'une induction plus tardive (DO = 63,5 contre 32,5) et moins forte (concentration en IAA égale à 5 mg/l contre 25 mg/l) permet de multiplier par un facteur 3 à 4 la quantité de protéine recombinante obtenue par unité de volume fermenté.

La souche E. *coli* ICONE 200, obtenue par modification génétique selon l'invention d'une souche d'intérêt industriel, permet de contrôler strictement l'expression de tout gène placé sur un vecteur plasmidique en aval du promoteur tryptophane Ptrp. Ce contrôle est transitoire car médié par le tryptophane exogène apporté à la culture. Le potentiel d'induction de Ptrp dans ICONE 200 est conservé et demeure modulable par la concentration d'IAA. Grâce à ces caractéristiques, ICONE 200 permet l'expression contrôlée de protéines recombinantes dans des conditions de culture extrapolables à grande échelle.

### Bibliographie

Aldabe, R., Barco, A. & Carrasco, L. (1996). The Journal of Biological Chemistry 271, 23134-23137.
Botsford, J.L. & DcMoss, R.D. (1971). Catabolite repression of tryptophanase in Escherichia *coli*. Journal of Bacteriology, 105, 303-312.
Deeley, M.C. & Yanofsky, C. (1981). Nucleotide sequence of the structural gene for tryptophanase of Escherichia *coli* K-12. Journal of Bacteriology, 147, 787-796.
Gunsalus, R.P., Yanofsky, C. (1980). Nucleotide sequence and expression of E. *coli* trpR, the structural gene for the trp aporepressor. Proceeding of the National Academy os Sciences, USA, 77, 12, 7117-7121.
Gunsalus, R.P., Gunsalus Miguel, A. & Gunsalus, G.L. (1986). Intracellular Trp repressor levels in Escherichia coli. Journal of Bacteriology, 167, 272-278.
Hamilton, C.M., Aldea, M., Washburn, B.K., Babitzke, P. & Kushner, S.R. (1989). New method for generating deletions and gene replacements in Escherichia *coli*. Journal of Bacteriology, 171, 4617-4622.
Hasan, N. & Szybalski, W. (1995). Construction of lacIts and lacIqts expression plasmids and evaluation of the thermosensitive lac repressor. Gene, 163, 35-40.
Hultman, T., Stahl, S., Hornes, E. & Uhlen, M. (1989). Direct solid phase sequencing of genomic and plasmid DNA using magnetic beads as solid support Nucleic Acids Research, 17, 4937-4946.
Isaacs, H., Chao, D., Yanofsky, C. & Saier, M.H. (1994). Mechanism of catabolite repression of tryptophanase synthesis in Escherichia *coli*. Microbiology, 140, 2125-2134.
Kelley, R.L. & Yanofsky, C. (1982). trp aporepressor production is controlled by autogenous regulation and inefficient translation. Proceedings of the National Academy of Sciences, USA, 79, 3120-3124.
Lama, J. & Carrasco, L. (1992). The Journal of Biological Chemistry 267, 15932-15937.
Maurer, R., Meyer, B.J. & Ptashne, M. (1980). Gene regulation at the right operator (OR) of bacteriophage lambda. 1. OR3 and autogenous negative control by repressor. Journal of Molecular Biology, 139, 147-161.
Murby, M., Samuelsson, E., Nguyen. T.N., Mignard, L., Power, U., Binz, H., Uhlen, M. & Stahl, S. (1995). Hydrophobicity engineering to increase solubility and stability of a recombinant protein from respiratory syncytial virus. European Journal of Biochemistry, 230, 38-44.
Nichols, B.P. & Yanofsky, C. (1983). Plasmids containing the trp promoters of Escherichia *coli* and Serratia *marcescens* and their use in expressing cloned genes. Methods in Enzymolosy, 101, 155-164.
Snell, E.E. (1975). Tryptophanase : structure, catalytic activities, and mechanism of action. Advances in Enzymology and Related Areas of Molecular Biology, 42, 287-333.
Stark, M.J.R. (1987). Multicopy expression vectors carrying the lac repressor gene for regulated high-level expression of genes in Escherichia *coli*. Gene, 51, 255-267.
Stewart, V. & Yanofsky, C. (1985). Evidence for transcription antitermination control of tryptophanase operon expression in Escherichia *coli* K-12. Journal of Bacteriology, 164, 731-740.
Suter-Crazzolara, C. & Unsicker, K. (1995). Improved expression of toxic proteins in E. *coli*. BioTechniques, 19, 202-204.
Yanofsky, C. et al. (1981). The complete nucléotide sequence of the tryptophan operon of E. *coli*. Nucleic Acids Research, 9, 24, 6647-6668.
Yansura, D.G. & Bass, S.H. (1997). Application of the E. *coli* trp promoter. Methods in Molecular Biology, 62, 55-62.
Yansura, D.G. & Henner, D.J. (1990). Use of Escherichia *coli* trp promoter for direct expression of proteins. In Anonymous, Methods in Enzymology. (pp. 54-60). San Diego, CA: Academic Press, Inc.

## Revendications

1. Procédé de production d'une protéine recombinante d'intérêt dont le gène est placé sous le contrôle du promoteur de l'opéron tryptophane Ptrp **caractérisé en ce qu'**il comprend les étapes suivantes :
a) la transformation d'une cellule procaryote par un vecteur contenant une séquence nucléique qui, lorsque ladite séquence nucléique est introduite dans ladite cellule hôte, modifie le gène codant pour la tryptophanase TnaA de la cellule hôte de telle sorte que cette modification entraîne la perte de l'activité tryptophanase de ladite cellule hôte, le produit d'expression dudit gène modifié étant incapable de dégrader le tryptophane en indole, et l'intégration de ladite séquence dans l'ADN de ladite cellule hôte ; et précédemment, postérieurement ou simultanément, l'introduction dans ladite cellule procaryote de tout, ou une partie, de la séquence d'un promoteur suivie en 3' d'une séquence nucléique codant pour une molécule de nature ribonucléotidique ou protéique agissant négativement sur le promoteur Ptrp ou son produit de transcription, ladite molécule de nature ribonucléotidique agissant négativement sur le promoteur Ptrp étant choisie parmi les séquences suivantes: 5' - AUUCGCGUCU ACGGCUUCAU CGUGUUGCGC - 3', 
5' - AUUCGCGUCU ACGGCUUCAU CGUGUUGCGC AGCACAACGC
GCCUGUCACC GGAUGUGUUU UCCGGUCUGA UGAGUCCGUG
AGGACGAAAC AGG - 3',
 
5' - AUUCAGUACG AAAAUUGCUU UCAUAAUUCU AGAUACCCUU
UUUACGUGAA CUU - 3',
 
5' - AUUCAGUACG AAAAUUGCUU UCAUAAUUCU AGAUACCCUU
UUUACGUGAA CUUAGCACAA CGCGCCUGUC ACCGGAUGUG
UUUUCCGGUC UGAUGAGUCC GUGAGGACGA AACAGG - 3',
 
5' - AUUCGCGUCU ACGGCUUCAU CGUGUUGCGC AUUCAGUACG
AAAAUUGCUU UCAUAAUUCU AGAUACCCUU UUUACGUGAA CUU - 3',
 
5' - AUUCGCGUCU ACGGCUUCAU CGUGUUGCGC AUUCAGUACG
AAAAUUGCUU UCAUAAUUCU AGAUACCCUU UUUACGUGAA
CUUAGCACAA CGCGCCUGUC ACCGGAUGUG UUUUCCGGUC
UGAUGAGUCC GUGAGGACGA AACAGG - 3',
 5' - CUUCGCGUCC UGAUGAGUCC GUGAGGACGA AACGGCUUCC - 3', 
5' - CUUCGCGUCC UGAUGAGUCC GUGAGGACGA AACGGCUUCC
AGCACAACGC GCCUGUCACC GGAUGUGUUU UCCGGUCUGA
UGAGUCCGUG AGGACGAAAC AGG - 3',
 ladite molécule de nature protéique agissant négativement sur le promoteur Ptrp étant choisie parmi la séquence codant pour l'aporépresseur TrpR de l'opéron tryptophane de ladite cellule hôte ou un de ses fragments biologiquement actifs ayant conservé son activité répresseur ;
b) la transformation de ladite cellule procaryote par un vecteur contenant un gène codant pour ladite protéine recombinante d'intérêt ;
c) la culture de ladite cellule transformée dans un milieu de culture permettant l'expression de la protéine recombinante ; et
d) la récupération de la protéine recombinante à partir du milieu de culture ou ladite cellule transformée.

2. Procédé de production d'une protéine recombinante d'intérêt selon la revendication 1, dans lequel la séquence nucléique capable d'inactiver le gène codant pour une tryptophanase TnaA est introduite dans l'ADN de la cellule hôte procaryote.

3. Procédé de production d'une protéine recombinante d'intérêt selon la revendication 1 ou 2, dans lequel ladite séquence nucléique introduite dans ladite cellule hôte est introduite sans autre élément d'ADN qui permettrait d'y associer un avantage sélectif.

4. Procédé de production d'une protéine recombinante d'intérêt selon l'une des revendications 1 à 3, dans lequel ladite séquence nucléique introduite dans ladite cellule hôte est introduite au locus de l'opéron tryptophanase.

5. Procédé de production d'une protéine recombinante d'intérêt selon l'une des revendications 1 à 4, **caractérisé en ce que** ledit procédé comprend en outre entre l'étape a) et b), une étape de résolution et de criblage.

6. Procédé de production selon l'une des revendications 1 à 5, dans lequel l'induction dudit promoteur suivi en 3' d'une séquence nucléique codant pour une molécule de nature ribonucléotidique ou protéique agissant négativement sur le promoteur Ptrp ou son produit de transcription est obtenue soit :
a) par le choix d'une source de carbone appropriée dans le milieu de culture ; soit
b) par l'ajout de tryptophane dans le milieu de culture ; ou
c) par une combinaison de a) et b).

7. Procédé selon l'une des revendications 1 à 6, **caractérisé en ce que** la cellule hôte procaryote est une bactérie Gram négative.

8. Procédé selon l'une des revendications 1 à 7, **caractérisé en ce que** la cellule hôte procaryote est *E. Coli*.

9. Construction pour l'expression d'un gène codant pour une protéine recombinante d'intérêt placé sous le contrôle du promoteur de l'opéron tryptophane Ptrp dans une cellule hôte procaryote,
- **caractérisée en ce que** la construction comprend une séquence nucléique qui, lorsque ladite séquence nucléique est introduite dans ladite cellule hôte, modifie le gène codant pour la tryptophanase TnaA de la cellule hôte de telle sorte que cette modification entraîne la perte de l'activité tryptophanase de ladite cellule hôte, le produit d'expression dudit gène modifié étant incapable de dégrader le tryptophane en indole, et
- **caractérisée en ce que** ladite séquence nucléique capable d'inactiver le gène codant pour une tryptophanase TnaA lorsque ladite séquence nucléique est introduite dans ladite cellule hôte est la séquence nucléique comprenant tout ou partie de la séquence d'un promoteur suivie en 3' d'une séquence nucléique codant pour une molécule de nature ribonucléotidique ou protéique agissant négativement sur le promoteur Ptrp ou son produit de transcription, ladite molécule de nature ribonucléotidique agissant négativement sur le promoteur Ptrp étant choisie parmi les séquences suivantes : 5' - AUUCGCGUCU ACGGCUUCAU CGUGUUGCGC - 3', 
5' - AUUCGCGUCU ACGGCUUCAU CGUGUUGCGC AGCACAACGC
GCCUGUCACC GGAUGUGUUU UCCGGUCUGA UGAGUCCGUG
AGGACGAAAC AGG - 3',
 
5' - AUUCAGUACG AAAAUUGCUU UCAUAAUUCU AGAUACCCUU
UUUACGUGAA CUU - 3',
 
5' - AUUCAGUACG AAAAUUGCUU UCAUAAUUCU AGAUACCCUU
UUUACGUGAA CUUAGCACAA CGCGCCUGUC ACCGGAUGUG
UUUUCCGGUC UGAUGAGUCC GUGAGGACGA AACAGG - 3',
 
5' - AUUCGCGUCU ACGGCUUCAU CGUGUUGCGC AUUCAGUACG
AAAAUUGCUU UCAUAAUUCU AGAUACCCUU UUUACGUGAA CUU - 3',
 
5' - AUUCGCGUCU ACGGCUUCAU CGUGUUGCGC AUUCAGUACG
AAAAUUGCUU UCAUAAUUCU AGAUACCCUU UUUACGUGAA
CUUAGCACAA CGCGCCUGUC ACCGGAUGUG UUUUCCGGUC
UGAUGAGUCC GUGAGGACGA AACAGG - 3',
 5' - CUUCGCGUCC UGAUGAGUCC GUGAGGACGA AACGGCUUCC - 3', 
5' - CUUCGCGUCC UGAUGAGUCC GUGAGGACGA AACGGCUUCC
AGCACAACGC GCCUGUCACC GGAUGUGUUU UCCGGUCUGA
UGAGUCCGUG AGGACGAAAC AGG - 3';
ladite molécule de nature protéique agissant négativement sur le promoteur Ptrp étant choisie parmi la séquence codant pour l'aporépresseur TrpR de l'opéron tryptophane de ladite cellule hôte ou un de ses fragments biologiquement actifs ayant conservé son activité répresseur.

10. Construction selon la revendication 9, **caractérisée en ce qu'**elle comprend en outre en amont de ladite séquence nucléique capable d'inactiver le gène codant pour une tryptophanase TnaA lorsque ladite séquence nucléique est introduite dans ladite cellule hôte, tout ou partie de la séquence nucléique du promoteur Ptna de l'opéron tryptophanase.

11. Construction selon la revendication 9 ou 10, **caractérisée en ce que** ladite séquence nucléique capable d'inactiver le gène codant pour une tryptophanase TnaA lorsque ladite séquence nucléique est introduite dans ladite cellule hôte comprend un fragment muté de la séquence codante de ladite tryptophanase TnaA..

12. Construction selon la revendication 11, **caractérisée en ce que** ledit fragment muté est obtenu par l'insertion d'un codon stop à une position telle que la séquence du fragment muté ainsi obtenu code pour un fragment protéique dépourvu d'activité tryptophanase.

13. Construction selon la revendication 11 ou 12, **caractérisée en ce que** ledit fragment muté est un fragment muté de la séquence codante de la tryptophanase TnaA de ladite cellule hôte.

14. Construction selon la revendication 9, **caractérisée en ce que** ledit promoteur suivi en 3' d'une séquence nucléique codant pour une molécule de nature ribonucléotidique ou protéique agissant négativement sur le promoteur Ptrp est tout, ou une partie permettant une activité promotrice, du promoteur Ptna de l'opéron tryptophanase.

15. Construction selon la revendication 14, **caractérisée en ce que** ladite séquence nucléique codant pour une molécule de nature ribonucléotidique ou protéique agissant négativement sur le promoteur Ptrp est la séquence codant pour l'aporépresseur TrpR de l'opéron tryptophane ou un de ses fragments biologiquement actifs.

16. Vecteur contenant une construction selon l'une des revendications 9 à 15.

17. Vecteur selon la revendication 16, **caractérisé en ce qu'**il s'agit du vecteur pMAK705 [tnaAt] ou du vecteur pMAK705 [Ptna : trpR : 3'tna],
- ledit vecteur pMAK705 [tnaAt] étant constitué du vecteur pMAK705 dont la séquence est identifiée dans GenBank sous le numéro d'accession AF519766, ce vecteur comprenant un fragment d'ADN du gène codant pour la tryptophanase TnaA, noté tnaAT, s'étendant des positions - 275 à + 1054 par rapport au premier nucléotide de la séquence codante de tnaA amplifié à l'aide des deux couples d'amorces Trp5 (sens)/Trp2 et Trp3 /Trp4 ; et
- ledit vecteur pMAK705 [pMAK705 [Ptna : trpR : 3'tna] constitué du vecteur pMAK705, ce vecteur comprenant trois sous-unités, la première sous-unité située dans le promoteur Ptna s'étendant des positions - 511 à + 3 par rapport au premier nucléotide de la séquence codante de tnaAet amplifiée à partir du couple d'amorce TrpR1/TrpR2, d'une seconde sous-unité correspond à la séquence codante du gène trpR d'E. coli et amplifiée à partir des amorces TrpR3/TrpR4, et d'une troisième sous-unité correspondant à la séquence située immédiatement en 3' de la séquence codante de tnaA et amplifiée à partir des amorcesTrpR5 /TrpR6,
ces couples d'amorce étant de séquence : Trp5 : 5' – CGGGATCCGTGTGACCTCAAAATGGTT - 3', et Trp2 : 3' – CTACGCGCCGCTGCTTCGGATTAGATCTCG - 5' ; Trp3 : 5' – CGTCTAGACAGCGGCAGTCGTAGCTAC - 3', et Trp4 : 3' – CCTTCTCTAACCGCAACAGTTCGAACG - 5' ; TrpR1 : 5' - CTGGATCCCTGTCAGATGCGCTTCGC - 3', et TrpR2 : 3' – CTTCCTAATACATTACCGGGTTG - 5' ; TrpR3 :     5' – GTAATGGCCCAACAATCACC - 3', et TrpR4 : 3' – CACAACGACTTTTCGCTAACTGACGTCAG - 5' ; TrpR5 :     5' – CGCTGCAGTTAATACTACAGAGTGG - 3', et

TrpR6 :     3' – CCAGCTAATGAGGTAAGTTCGAAC - 5'.

18. Cellule hôte procaryote transformée par un vecteur selon l'une des revendications 16 à 17.

19. Cellule hôte procaryote selon la revendication 18, **caractérisée en ce qu'**il s'agit d'*E*. *coli*.

20. Utilisation d'une bactérie transformée par un secteur selon l'une des revendications 16 et 17 pour la production de protéines recombinantes.

## Claims

1. Method for producing a recombinant protein of interest, the gene of which is placed under the control of the Ptrp tryptophan operon promoter, **characterized in that** it comprises the following steps:
a) transforming a prokaryotic cell with a vector containing a nucleic acid sequence which, when said nucleic acid sequence is introduced into said host cell, modifies the gene encoding the TnaA tryptophanase of the host cell, such that this modification leads to the loss of tryptophanase activity of said host cell, the product of expression of said modified gene being incapable of degrading tryptophan to indole, and the integration of said sequence into the DNA of said host cell; and, beforehand, subsequently or simultaneously, introducing into said prokaryotic cell all or part of the sequence of a promoter followed, in the 3' position, by a nucleic acid sequence encoding a molecule which is ribonucleotide or protein in nature, and which acts negatively on the Ptrp promoter or its transcription product, said molecule which is ribonucleotide in nature, and which acts negatively on the Ptrp promoter, being chosen from the following sequences: 5' - AUUCGCGUCU ACGGCUUCAU CGUGUUGCGC-3', 
5' - AUUCGCGUCU ACGGCUUCAU CGUGUUGCGC AGCACAACGC
GCCUGUCACC GGAUGUGUUU UCCGGUCUGA UGAGUCCGUG
AGGACGAAAC AGG-3',
 
5′ - AUUCAGUACG AAAAUUGCUU UCAUAAUUCU AGAUACCCUU
UUUACGUGAA CUU-3′,
 
5′ - AUUCAGUACG AAAAUUGCUU UCAUAAUUCU AGAUACCCUU
UUUACGUGAA CUUAGCACAA CGCGCCUGUC ACCGGAUGUG
UUUUCCGGUC UGAUGAGUCC GUGAGGACGA AACAGG-3′,
 
5′ - AUUCGCGUCU ACGGCUUCAU CGUGUUGCGC AUUCAGUACG
AAAAUUGCUU UCAUAAUUCU AGAUACCCUU UUUACGUGAA
CUU-3′,
 
5′ - AUUCGCGUCU ACGGCUUCAU CGUGUUGCGC AUUCAGUACG
AAAAUUGCUU UCAUAAUUCU AGAUACCCUU UUUACGUGAA
CUUAGCACAA CGCGCCUGUC ACCGGAUGUG UUUUCCGGUC
UGAUGAGUCC GUGAGGACGA AACAGG-3′,
 
5′ - CUUCGCGUCC UGAUGAGUCC GUGAGGACGA AACGGCUUCC-
3′,
 and 
5' - CUUCGCGUCC UGAUGAGUCC GUGAGGACGA AACGGCUUCC
AGCACAACGC GCCUGUCACC GGAUGUGUUU UCCGGUCUGA
UGAGUCCGUG AGGACGAAAC AGG-3'
 said molecule which is protein in nature, and which acts negatively on the Ptrp promoter, being chosen from the sequence encoding the TrpR tryptophan operon aporepressor of said host cell or one of its biologically active fragments that has conserved its repressor activity;
b) transforming said prokaryotic cell with a vector containing a gene encoding said recombinant protein of interest;
c) culturing said transformed cell in a culture medium which allows the expression of the recombinant protein; and
d) recovering the recombinant protein from the culture medium or from said transformed cell.

2. Method for producing a recombinant protein of interest according to Claim 1, in which the nucleic acid sequence capable of inactivating the gene encoding a TnaA tryptophanase is introduced into the DNA of the prokaryotic host cell.

3. Method for producing a recombinant protein of interest according to Claim 1 or 2, in which said nucleic acid sequence introduced into said host cell is introduced without any other DNA element which would allow a selective advantage to be associated therewith.

4. Method for producing a recombinant protein of interest according to one of Claims 1 to 3, in which said nucleic acid sequence introduced into said host cell is introduced at the tryptophanase operon locus.

5. Method for producing a recombinant protein of interest according to one of Claims 1 to 4, **characterized in that** said method also comprises, between step a) and b), a resolution and a screening step.

6. Production method according to one of Claims 1 to 5, in which the induction of said promoter which is followed, in the 3' position, by a nucleic acid sequence encoding a molecule which is ribonucleotide or protein in nature, and which acts negatively on the Ptrp promoter or its transcription product is obtained either:
a) by choosing a suitable carbon source in the culture medium; or
b) by adding tryptophan to the culture medium; or
c) by a combination of a) and b).

7. Method according to one of Claims 1 to 6, **characterized in that** the prokaryotic host cell is a Gram-negative bacterium.

8. Method according to one of Claims 1 to 7, **characterized in that** the prokaryotic host cell is E. *coli*.

9. Construct for expressing a gene encoding a recombinant protein of interest placed under the control of the Ptrp tryptophan operon promoter in a prokaryotic host cell, **characterized in that** the construct comprises a nucleic acid sequence which, when said nucleic acid sequence is introduced into said host cell, modifies the gene encoding the TnaA tryptophanase of the host cell, such that this modification leads to the loss of the tryptophanase activity of said host cell, the product of expression of said modified gene being incapable of degrading tryptophan to indole, and **characterized in that** said nucleic acid sequence capable of inactivating the gene encoding a TnaA tryptophanase when said nucleic acid sequence is introduced into said host cell is the nucleic acid sequence comprising all or part of the sequence of a promoter followed, in the 3' position, by a nucleic acid sequence encoding a molecule which is ribonucleotide or protein in nature, and which acts negatively on the Ptrp promoter or its transcription product, said molecule which is ribonucleotide in nature, and which acts negatively on the Ptrp promoter, being chosen from the following sequences: 5' - AUUCGCGUCU ACGGCUUCAU CGUGUUGCGC-3', 
5′ - AUUCGCGUCU ACGGCUUCAU CGUGUUGCGC AGCACAACGC
GCCUGUCACC GGAUGUGUUU UCCGGUCUGA UGAGUCCGUG AGGACGAAAC
AGG-3′,
 
5' - AUUCAGUACG AAAAUUGCUU UCAUAAUUCU AGAUACCCUU
UUUACGUGAA CUU-3',
 
5' - AUUCAGUACG AAAAUUGCUU UCAUAAUUCU AGAUACCCUU
UUUACGUGAA CUUAGCACAA CGCGCCUGUC ACCGGAUGUG UUUUCCGGUC
UGAUGAGUCC GUGAGGACGA AACAGG-3',
 
5' - AUUCGCGUCU ACGGCUUCAU CGUGUUGCGC AUUCAGUACG
AAAAUUGCUU UCAUAAUUCU AGAUACCCUU UUUACGUGAA CUU-3',
 
5' - AUUCGCGUCU ACGGCUUCAU CGUGUUGCGC AUUCAGUACG
AAAAUUGCUU UCAUAAUUCU AGAUACCCUU UUUACGUGAA CUUAGCACAA
CGCGCCUGUC ACCGGAUGUG UUUUCCGGUC UGAUGAGUCC GUGAGGACGA
AACAGG-3',
 5' - CUUCGCGUCC UGAUGAGUCC GUGAGGACGA AACGGCUUCC-3', and 
5′ - CUUCGCGUCC UGAUGAGUCC GUGAGGACGA AACGGCUUCC
AGCACAACGC GCCUGUCACC GGAUGUGUUU UCCGGUCUGA UGAGUCCGUG
AGGACGAAAC AGG-3′;
 said molecule which is protein in nature, and which acts negatively on the Ptrp promoter, being chosen from the sequence encoding the TrpR tryptophan operon aporepressor of said host cell or one of its biologically active fragments that has conserved its repressor activity.

10. Construct according to Claim 9, **characterized in that** it also comprises, upstream of said nucleic acid sequence capable of inactivating the gene encoding a TnaA tryptophanase when said nucleic acid sequence is introduced into said host cell, all or part of the nucleic acid sequence of the Ptna tryptophanase operon promoter.

11. Construct according to Claim 9 or 10, **characterized in that** said nucleic acid sequence capable of inactivating the gene encoding a TnaA tryptophanase when said nucleic acid sequence is introduced into said host cell comprises a mutated fragment of the coding sequence of said TnaA tryptophanase.

12. Construct according to Claim 11, **characterized in that** said mutated fragment is obtained by inserting a stop codon at a position such that the sequence of the mutated fragment thus obtained encodes a protein fragment lacking tryptophanase activity.

13. Construct according to either of Claims 11 and 12, **characterized in that** said mutated fragment is a mutated fragment of the coding sequence of the TnaA tryptophanase of said host cell.

14. Construct according to Claim 9, **characterized in that** said promoter followed, in the 3' position, by a nucleic acid sequence encoding a molecule which is ribonucleotide or protein in nature, and which acts negatively on the Ptrp promoter, is all, or a part allowing promoter activity, of the Ptna tryptophanase operon promoter.

15. Construct according to Claim 14, **characterized in that** said nucleic acid sequence encoding a molecule which is ribonucleotide or protein in nature, and which acts negatively on the Ptrp promoter, is the sequence encoding the TrpR tryptophan operon aporepressor or one of its biologically active fragments.

16. Vector containing a construct according to one of Claims 9 to 15.

17. Vector according to Claim 16, **characterized in that** it is the vector pMAK705[tnaAt] or the vector pMAK705[Ptna : trpR : 3'tna],
- said vector pMAK705 [tnaAt] consisting of the vector pMAK705, the sequence of which is identified in GenBank under the accession number AF519766, this vector comprising a fragment of DNA of the gene encoding the TnaA tryptophanase, referred to as tnaAT, extending from positions -275 to +1054 relative to the first nucleotide of the coding sequence of tnaA amplified using the two pairs of primers Trp5 (sense)/Trp2 and Trp3/Trp4; and
- said vector pMAK705 [Ptna : trpR : 3'tna] consisting of the vector pMAK705, this vector comprising three subunits, the first subunit located in the Ptna promoter extending from positions -511 to +3 relative to the first nucleotide of the coding sequence of tnaA and amplified using the pair of primers TrpR1/TrpR2, a second subunit corresponding to the coding sequence of the E. coli trpR gene and amplified using the primers TrpR3/TrpR4, and a third subunit corresponding to the sequence located immediately 3' of the coding sequence of tnaA and amplified using the primers TrpR5/TrpR6,
these pairs of primers having the sequence: Trp5: 5' - CGGGATCCGTGTGACCTCAAAATGGTT - 3', and Trp2: 3' - CTACGCGCCGCTGCTTCGGATTAGATCTCG - 5'; Trp3: 5' - CGTCTAGACAGCGGCAGTCGTAGCTAC - 3' and Trp4: 3' - CCTTCTCTAACCGCAACAGTTCGAACG - 5'; TrpR1: 5' - CTGGATCCCTGTCAGATGCGCTTCGC - 3' and TrpR2: 3' - CTTCCTAATACATTACCGGGTTG - 5'; TrpR3: 5' - GTAATGGCCCAACAATCACC - 3', and TrpR4: 3' - CACAACGACTTTTCGCTAACTGACGTCAG - 5'; TrpR5: 5' - CGCTGCAGTTAATACTACAGAGTGG - 3', and

TrpR6: 3' - CCAGCTAATGAGGTAAGTTCGAAC - 5'.

18. Prokaryotic host cell transformed with a vector according to either of Claims 16 and 17.

19. Prokaryotic host cell according to Claim 18, **characterized in that** it is E. *coli*.

20. Use of a bacterium transformed with a vector according to either of Claims 16 and 17, for producing recombinant proteins.

## Patentansprüche

1. Verfahren zur Herstellung eines rekombinanten Proteins von Interesse, dessen Gen unter die Kontrolle des Promotors des Tryptophan-Operons Ptrp gestellt ist, **dadurch gekennzeichnet, dass** es die folgenden Schritte umfasst:
a) die Transformation einer prokaryotischen Zelle durch einen Vektor, der eine Nukleinsäuresequenz enthält, die, wenn die Nukleinsäuresequenz in die Wirtszelle eingeführt wird, das die Tryptophanase TnaA kodierende Gen der Wirtszelle derart modifiziert, dass diese Modifikation den Verlust der Tryptophanase-Aktivität der Wirtszelle zur Folge hat, wobei das Expressionsprodukt des modifizierten Gens nicht in der Lage ist, Tryptophan zu Indol abzubauen, und die Integration der Sequenz in die DNA der Wirtszelle; und zuvor, später oder gleichzeitig die Einführung in die prokaryotische Wirtszelle der Gesamtheit oder eines Teils der Sequenz eines Promotors, gefolgt 3' von einer Nukleinsäuresequenz, welche ein Molekül von Ribonukleotid- oder Protein-Natur kodiert, welches auf den Ptrp-Promotor oder dessen Transkriptionsprodukt negativ einwirkt, wobei das Molekül von Ribonukleotid-Natur, welches auf den Ptrp-Promotor negativ einwirkt, ausgewählt wird aus den folgenden Sequenzen: 5' -AUUCGCGUCU ACGGCUUCAU CGUGUUGCGC - 3', 
5' -AUUCGCGUCU ACGGCUUCAU CGUGUUGCGC AGCACAACGC
GCCUGUCACC GGAUGUGUUU UCCGGUCUGA UGAGUCCGUG
AGGACGAAAC AGG - 3',
 
5' - AUUCAGUACG AAAAUUGCUU UCAUAAUUCU AGAUACCCUU
UUUACGUGAA CUU - 3',
 
5' - AUUCAGUACG AAAAUUGCUU UCAUAAUUCU AGAUACCCUU
UUUACGUGAA CUUAGCACAA CGCGCCUGUC ACCGGAUGUG
UUUUCCGGUC UGAUGAGUCC GUGAGGACGA AACAGG - 3',
 
5' - AUUCGCGUCU ACGGCUUCAU CGUGUUGCGC AUUCAGUACG
AAAAUUGCUU UCAUAAUUCU AGAUACCCUU UUUACGUGAA CUU - 3',
 
5' - AUUCGCGUCU ACGGCUUCAU CGUGUUGCGC AUUCAGUACG
AAAAUUGCUU UCAUAAUUCU AGAUACCCUU UUUACGUGAA
CUUAGCACAA CGCGCCUGUC ACCGGAUGUG UUUUCCGGUC
UGAUGAGUCC GUGAGGACGA AACAGG - 3',
 5' - CUUCGCGUCC UGAUGAGUCC GUGAGGACGA AACGGCUUCC - 3', und 
5' - CUUCGCGUCC UGAUGAGUCC GUGAGGACGA AACGGCUUCC
AGCACAACGC GCCUGUCACC GGAUGUGUUU UCCGGUCUGA
UGAGUCCGUG AGGACGAAAC AGG - 3',
wobei das Molekül von Protein-Natur, welches auf den Ptrp-Promotor negativ einwirkt, ausgewählt wird aus der Sequenz, welche den Aporepressor TrpR des Tryptophan-Operons der Wirtszelle oder eines von dessen biologisch aktiven Fragmenten, bei welchem dessen Repressor-Aktivität erhalten geblieben ist, kodiert;
b) die Transformation der prokaryotischen Zelle durch einen Vektor, welcher ein Gen enthält, welches das rekombinante Protein von Interesse kodiert;
c) die Kultivierung der transformierten Zelle in einem Kulturmedium, welches die Expression des rekombinanten Proteins erlaubt; und
d) die Gewinnung des rekombinanten Proteins ausgehend von dem Kulturmedium oder der transformierten Zelle.

2. Verfahren zur Herstellung eines rekombinanten Proteins von Interesse nach Anspruch 1, in welchem die Nukleinsäuresequenz, welche in der Lage ist, das eine Tryptophanase TnaA kodierende Gen zu inaktivieren, in die DNA der prokaryotischen Wirtszelle eingeführt wird.

3. Verfahren zur Herstellung eines rekombinanten Proteins von Interesse nach Anspruch 1 oder 2, in welchem die Nukleinsäuresequenz, die in die Wirtszelle eingeführt wird, ohne ein anderes DNA-Element eingeführt wird, welches erlauben würde, damit einen Selektionsvorteil zu verbinden.

4. Verfahren zur Herstellung eines rekombinanten Proteins von Interesse nach einem der Ansprüche 1 bis 3, in welchem die in die Wirtszelle eingeführte Nukleinsäuresequenz an dem Locus des Tryptophanase-Operons eingeführt wird.

5. Verfahren zur Herstellung eines rekombinanten Proteins von Interesse nach einem der Ansprüche 1 bis 4, **dadurch gekennzeichnet, dass** das Verfahren außerdem zwischen dem Schritt a) und b) einen Schritt einer Auftrennung und eines Screenings umfasst.

6. Herstellungsverfahren nach einem der Ansprüche 1 bis 5, in welchem die Induktion des Promotors, welcher 3' gefolgt wird von einer Nukleinsäuresequenz, welche ein Molekül von Ribonukleotid- oder Protein-Natur kodiert, welches auf den Ptrp-Promotor oder dessen Transkriptionsprodukt negativ einwirkt, erhalten wird entweder:
a) durch die Wahl einer geeigneten Kohlenstoffquelle in dem Kulturmedium; oder
b) durch das Hinzufügen von Tryptophan in das Kulturmedium; oder
c) durch eine Kombination von a) und b).

7. Verfahren nach einem der Ansprüche 1 bis 6, **dadurch gekennzeichnet, dass** die prokaryotische Wirtszelle ein gramnegatives Bakterium ist.

8. Verfahren nach einem der Ansprüche 1 bis 7, **dadurch gekennzeichnet, dass** die prokaryotische Wirtszelle *E. coli* ist.

9. Konstrukt für die Expression eines ein rekombinantes Protein von Interesse kodierenden Gens, welches unter die Kontrolle des Promotors des Tryptophan-Operons Ptrp gestellt ist, in einer prokaryotischen Wirtszelle,
- **dadurch gekennzeichnet, dass** das Konstrukt eine Nukleinsäuresequenz umfasst, die, wenn die Nukleinsäuresequenz in die Wirtszelle eingeführt wird, das die Tryptophanase TnaA der Wirtszelle kodierende Gen derart modifiziert, dass diese Modifikation den Verlust der Tryptophanase-Aktivität der Wirtszelle zur Folge hat, wobei das Expressionsprodukt des modifizierten Gens nicht in der Lage ist, Tryptophan zu Indol abzubauen, und
- **dadurch gekennzeichnet, dass** die Nukleinsäuresequenz, die in der Lage ist, das eine Tryptophanase TnaA kodierende Gen zu inaktivieren, wenn die Nukleinsäuresequenz in die Wirtszelle eingeführt wird, die Nukleinsäuresequenz ist, welche die Gesamtheit oder einen Teil der Sequenz eines Promotors, gefolgt 3' von einer Nukleinsäuresequenz, welche ein Molekül von Ribonukleotid- oder Protein-Natur kodiert, welches auf den Ptrp-Promotor oder dessen Transkriptionsprodukt negativ einwirkt, umfasst, wobei das Molekül von Ribonukleotid-Natur, welches auf den Ptrp-Promotor negativ einwirkt, ausgewählt wird aus den folgenden Sequenzen: 5' - AUUCGCGUCU ACGGCUUCAU CGUGUUGCGC - 3', 
5' - AUUCGCGUCU ACGGCUUCAU CGUGUUGCGC AGCACAACGC
GCCUGUCACC GGAUGUGUUU UCCGGUCUGA UGAGUCCGUG
AGGACGAAAC AGG - 3',
 
5' - AUUCAGUACG AAAAUUGCUU UCAUAAUUCU AGAUACCCUU
UUUACGUGAA CUU - 3',
 
5' - AUUCAGUACG AAAAUUGCUU UCAUAAUUCU AGAUACCCUU
UUUACGUGAA CUUAGCACAA CGCGCCUGUC ACCGGAUGUG
UUUUCCGGUC UGAUGAGUCC GUGAGGACGA AACAGG - 3',
 
5' - AUUCGCGUCU ACGGCUUCAU CGUGUUGCGC AUUCAGUACG
AAAAUUGCUU UCAUAAUUCU AGAUACCCUU UUUACGUGAA CUU - 3',
 
5' - AUUCGCGUCU ACGGCUUCAU CGUGUUGCGC AUUCAGUACG
AAAAUUGCUU UCAUAAUUCU AGAUACCCUU UUUACGUGAA
CUUAGCACAA CGCGCCUGUC ACCGGAUGUG UUUUCCGGUC
UGAUGAGUCC GUGAGGACGA AACAGG - 3',
 5' - CUUCGCGUCC UGAUGAGUCC GUGAGGACGA AACGGCUUCC - 3', und 
5' - CUUCGCGUCC UGAUGAGUCC GUGAGGACGA AACGGCUUCC
AGCACAACGC GCCUGUCACC GGAUGUGUUU UCCGGUCUGA
UGAGUCCGUG AGGACGAAAC AGG - 3';
wobei das Molekül von Protein-Natur, welches auf den Ptrp-Promotor negativ einwirkt, ausgewählt wird aus der Sequenz, welche den Aporepressor TrpR des Tryptophan-Operons der Wirtszelle oder eines von dessen biologisch aktiven Fragmenten, bei welchem dessen Repressor-AKtivität erhalten geblieben ist, kodiert.

10. Konstrukt nach Anspruch 9, **dadurch gekennzeichnet, dass** es außerdem strangaufwärts von der Nukleinsäuresequenz, welche in der Lage ist, das eine Tryptophanase TnaA kodierende Gen zu inaktivieren, wenn die Nukleinsäuresequenz in die Wirtszelle eingeführt wird, die Gesamtheit oder einen Teil der Nukleinsäuresequenz des Ptna-Promotors des Tryptophanase-Operons umfasst.

11. Konstrukt nach Anspruch 9 oder 10, **dadurch gekennzeichnet, dass** die Nukleinsäuresequenz, welche in der Lage ist, das eine Tryptophanase TnaA kodierende Gen zu inaktivieren, wenn die Nukleinsäuresequenz in die Wirtszelle eingeführt wird, ein mutiertes Fragment der die Tryptophanase TnaA kodierenden Sequenz umfasst.

12. Konstrukt nach Anspruch 11, **dadurch gekennzeichnet, dass** das mutierte Fragment erhalten wird durch Insertion eines Stop-Codons an einer solchen Position, dass die Sequenz des so erhaltenen mutierten Fragments ein Proteinfragment kodiert, welchem Tryptophanase-Aktivität fehlt.

13. Konstrukt nach Anspruch 11 oder 12, **dadurch gekennzeichnet, dass** das mutierte Fragment ein mutiertes Fragment der die Tryptophanase TnaA der Wirtszelle kodierenden Sequenz ist.

14. Konstrukt nach Anspruch 9, **dadurch gekennzeichnet, dass** der Promotor, welcher 3' gefolgt wird von einer Nukleinsäuresequenz, welche ein Molekül von Ribonukleotid- oder Protein-Natur kodiert, welches auf den Ptrp-Promotor negativ einwirkt, die Gesamtheit oder ein Teil, welcher eine Promotoraktivität erlaubt, des Promotors Ptna des Tryptophanase-Operons ist.

15. Konstrukt nach Anspruch 14, **dadurch gekennzeichnet, dass** die Nukleinsäuresequenz, welche ein Molekül von Ribonukleotid- oder Protein-Natur kodiert, welches auf den Ptrp-Promotor negativ einwirkt, die Sequenz ist, welche den Aporepressor TrpR des Tryptophan-Operons oder eines von dessen biologisch aktiven Fragmenten kodiert.

16. Vektor, welcher ein Konstrukt nach einem der Ansprüche 9 bis 15 enthält.

17. Vektor nach Anspruch 16, **dadurch gekennzeichnet, dass** es sich um den Vektor pMAK705 [tnaAt] oder den Vektor pMAK705 [Ptna : trpR : 3'tna] handelt,
- wobei der Vektor pMAK705 [tnaAt] aus dem Vektor pMAK705 gebildet wird, dessen Sequenz in GenBank unter der Aufnahmenummer AF519766 angegeben ist, wobei dieser Vektor ein DNA-Fragment des die Tryptophanase TnaA kodierenden Gens, welches als tnaAT bezeichnet wird, welches sich von den Positionen -275 bis +1054 bezogen auf das erste Nukleotid der kodierenden Sequenz von tnaA erstreckt, welches mit Hilfe der beiden Primerpaare Trp5(Sinn)/Trp2 und Trp3/Trp4 amplifiziert wird, umfasst; und
- wobei der Vektor pMAK705 [pMAK705 [Ptna : trpR : 3'tna] aus dem Vektor pMAK705 gebildet wird, wobei dieser Vektor drei Untereinheiten umfasst, wobei die erste Untereinheit sich in dem Prornotor Ptna befindet, sich von den Positionen -511 bis +3 bezogen auf das erste Nukleotid der kodierenden Sequenz von tnaA erstreckt und ausgehend von dem Primerpaar TrpR1/TrpR2 amplifiziert wird, eine zweite Untereinheit der kodierenden Sequenz des Gens trpR von E. coli, und welche ausgehend von den Primem TrpR3/TrpR4 amplifiziert wird, entspricht und eine dritte Untereinheit, welche der unmittelbar 3' von der kodierenden Sequenz von tnaA gelegenen Sequenz entspricht und ausgehend von den Primem TrpR5/TrpR6 amplifiziert wird,
wobei diese Primerpaare die folgenden Sequenzen haben: Trp5 : 5' – CGGGATCCGTGTGACCTCAAAATGGTT - 3', und Trp2 : 3' – CTACGCGCCGCTGCTTCGGATTAGATCTCG - 5'; Trp3 : 5'-CGTCTAGACAGCGGCAGTCGTAGCTAC-3', und Trp4 : 3'-CCTTCTCTAACCGCAACAGTTCGAACG-5'; TrpR1 : 5' - CTGGATCCCTGTCAGATGCGCTTCGC - 3', und TrpR2 : 3'-CTTCCTAATACATTACCGGGTTG-5'; TrpR3 : 5'-GTAATGGCCCAACAATCACC-3', und TrpR4 : 3'-CACAACGACTTTTCGCTAACTGACGTCAG-5'; TrpR5 : 5'-CGCTGCAGTTAATACTACAGAGTGG-3', und

TrpR6 : 3'-CCAGCTAATGAGGTAAGTTCGAAC-5'.

18. Prokaryotische Wirtszelle, die durch einen Vektor nach einem der Ansprüche 16 bis 17 transformiert ist.

19. Prokaryotische Wirtszelle nach Anspruch 18, **dadurch gekennzeichnet, dass** es sich um *E. coli* handelt.

20. Verwendung eines durch einen Vektor nach einem der Ansprüche 16 und 17 transformierten Bakteriums für die Herstellung von rekombinanten Proteinen.
